# EUROPÄISCHE PATENTSCHRIFT

(11) **EP 1 768 981 B1**
(45) Veröffentlichungstag und Bekanntmachung des Hinweises auf die Patenterteilung: **12.09.2012**
(21) Anmeldenummer: 05757944.3
(22) Anmeldetag: 07.07.2005
(51) Int. Cl.: C07D 471/04, A61K 31/4985, A61P 35/00

(54) **NEUE PYRIDODIHYDROPYRAZINONE, VERFAHREN ZU IHRER HERSTELLUNG UND IHRE VERWENDUNG ALS ARZNEIMITTEL**
NOVEL PYRIDODIHYDROPYRAZINONES METHOD FOR PRODUCTION AND USE THEREOF AS MEDICAMENTS
NOUVEAUX PYRIDODIHYDROPYRAZINONES, PROCEDE DE PRODUCTION ET UTILISATION DE CES DERNIERS COMME MEDICAMENTS

(30) Priorität: 09.07.2004 DE 102004033670
(43) Veröffentlichungstag der Anmeldung: 04.04.2007
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: HOFFMANN, Matthias, 88441 Mittelbiberach (DE); GRAUERT, Matthias, 88400 Biberach (DE); BRANDL, Trixi, 4056 Basel (DE); HAUPTMANN, Rudolf, A-2483 Ebreichsdorf (AT); STEEGMAIER, Martin, 72762 Reutlingen (DE)
(74) Vertreter: Hammann, Heinz
(86) Internationale Anmeldenummer: PCT/EP2005/007347
(87) Internationale Veröffentlichungsnummer: WO 2006/005510

(56) Entgegenhaltungen:
- WO-A-01/19825
- WO-A-02/076954
- WO-A-02/076985
- WO-A-03/020722
- WO-A-2004/076454
- US-A1- 2004 176 380

## Beschreibung

Die vorliegende Erfindung betrifft neue Pyridodihydropyrazinone, Verfahren zu ihrer Herstellung sowie ihre Verwendung als Arzneimittel. Die erfindungsgemäßen Verbindungen entsprechen der allgemeinen Formel (I), 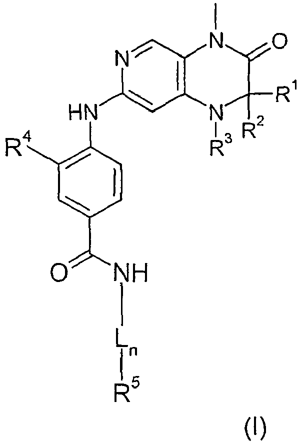 wobei die Reste L, R¹, R², R³, R⁴ und R⁵ die in den Ansprüchen und der Beschreibung genannten Bedeutungen haben.

### Hintergrund der Erfindung

Dihydro-pteridinon-Derivate (WO 03/020722) und Pyrido[3,4-b]pyrazinones (WO 2002/076954) sind als Wirkstoffe mit antiproliferativer Wirkung aus dem Stand der Technik bekannt.

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).

Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu et al. 2000, Cell 102:279-91). Ein spezifischer Zellzyklusarrest in der G2/M Phase kann aber auch z.B. durch Inhibition von spezifischen Phosphatasen wie z.B. Cdc25C (Russell and Nurse 1986, Cell 45:145-53) ausgelöst werden. Hefen mit defektem Cdc25 Gen arretieren in der G2 Phase, während eine Überexpression von Cdc25 zu einem verfrühten Eintritt in die Mitosephase führt (Russell und Nurse 1987, Cell 49:559-67). Desweiteren kann ein Arrest in der G2/M Phase auch durch Inhibition von bestimmten Motorproteinen, den so genannten Kinesinen wie z.B. Eg5 (Mayer et al. 1999, Science 286:971-4), oder durch Mikrotubuli stabilisierende oder destabilisierende Agentien (z.B. Colchicin, Taxol, Etoposid, Vinblastin, Vincristin) ausgelöst werden (Schiff und Horwitz 1980, Proc Natl Acad Sci U S A 77:1561-5).

Neben den Cyclin-abhängigen und den Aurora Kinasen spielen des weiteren die so genannten Polo-like Kinasen, eine kleine Familie von Serin/Threonin-Kinasen, eine wichtige Rolle bei der Regulation des eukaryontischen Zellzykluses. Bisher wurden die Polo-like Kinasen PLK-1, PLK-2, PLK-3 und PLK-4 in der Literatur beschrieben. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al. 1998, Genes Dev. 12:3777-87; Qian et al. 2001, Mol Biol Cell. 12:1791-9). Die Injektion von PLK-1 Antikörpern führt zu einem G2 Arrest in nicht transformierten Zellen, während Tumorzellen in der Mitosephase arretieren (Lane und Nigg 1996, J Cell Diol 135:1701-13). Überexpression von PLK-1 konnte für verschiedene Tumorarten, wie nicht kleinzelliges Lungenkarzinom, Plattenepithelkarzinom, Brust- und kolorektales Karzinom (Wolf et al. 1997, Oncogene 14 :543 -549; Knecht etal. 1999, Cancer Res. 59:2794 -2797; Wolf et al. 2000, Pathol. Res. Pract. 196:753 -759; Takahashi et al. 2003, Cancer Sci. 94:148-52) gezeigt werden. Daher stellt diese Klasse von Proteinen ebenfalls einen interessanten Angriffspunkt zur therapeutischen Intervention proliferativer Krankheiten dar (Liu and Erikson 2003, Proc Natl Acad Sci U S A 100:5789-5794).

Die Resistenz vieler Tumorarten erfordert die Entwicklung neuer Arzneimittel zur Tumorbekämpfung.

Es ist die Aufgabe der vorliegenden Erfindung neue Verbindungen mit antiproliferativer Wirkung bereitzustellen.

### Detaillierte Beschreibung der Erfindung

Überraschenderweise wurde gefunden, dass Verbindungen der allgemeinen Formel (I), worin die Reste L und R¹ bis R⁵ die nachstehend genannten Bedeutungen haben, als Inhibitoren spezifischer Zellzykluskinasen wirken. Die genannten Verbindungen zeigen antiproliferative Wirkung, indem sie Zellen in der Mitosephase des Zellzykluses arretieren bevor der programmierte Zelltod in den arretierten Zellen eingeleitet wird. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

(A1) Die vorliegende Erfindung betrifft daher Verbindungen der allgemeinen Formel (I) 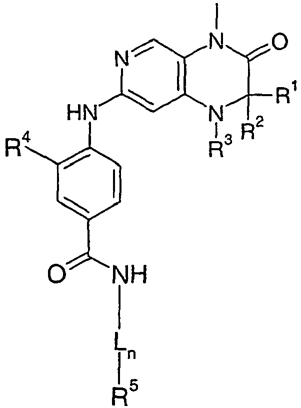 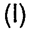 worin
R¹, R² gleich oder verschieden, Wasserstoff oder ein Rest, ausgewählt aus der Gruppe bestehend aus gegebenenfalls substituiertem C₁-C₆-Alkyl, C₂-C₆-Alkenyl, und C₂-C₆-Alkinyl,
oder
R¹ und R² gemeinsam eine 2- bis 5-gliedrige Alkylbrücke,
R³ Wasserstoff oder ein Rest, ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktem C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl,
R⁴ ein Rest, ausgewählt aus der Gruppe bestehend aus Wasserstoff, Hydroxy und Halogen, oder
ein Rest ausgewählt aus der Gruppe bestehend aus gegebenenfalls substituiertem C₁-C₃-Alkyl, C₂-C₆-Alkenyl, C₂-C₆-Alkinyl, C₁-C₅-Alkyloxy, C₂-C₅-Alkenyloxy und C₂-C₅-Alkinyloxy,
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₁-C₄-Alkyl-C₆-C₁₄-Aryl, C₆-C₁₄-Heteroaryl, und gegebenenfalls verbrücktem C₃-C₁₂-Cycloalkyl,
n 0 oder 1,
R⁵ ein Rest, ausgewählt aus der Gruppe bestehend aus Wasserstoff oder gegebenenfalls substituiertem C₁-C₆-Alkyl, C₁-C₆-Alkenyl, C₁-C₆-Alkinyl, C₃-C₁₂-Cycloalkyl, oder
ein Rest, ausgewählt aus der Gruppe bestehend aus gegebenenfalls substituiertem Pyridyl, Morpholinyl, Piperidinyl, Piperazinyl, Piperazinylcarbonyl, Pyrrolidinyl, Tropenyl, Sulfoxomorpholinyl, Sulfonylmorpholinyl, Thiomorpholinyl und Azacycloheptyl,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate oder Hydrate, vorzugsweise Mono- oder Dihydrate,
bedeuten.
Bevorzugt sind Verbindungen der Formel (I), worin
R³ bis R⁵, n und L die angegebene Bedeutung aufweisen, und
R¹, R² gleich oder verschieden, Wasserstoff , oder
ein Rest ausgewählt aus der Gruppe bestehend Methyl, Ethyl, Propyl, Propargyl und Allyl,
oder
R¹ und R² gemeinsam Cyclopropyl,
bedeuten.
Weiterhin bevorzugt sind Verbindungen der Formel (I), worin
R¹ bis R⁴ , n und L die angegebene Bedeutung aufweisen, und
R⁵ ein Rest ausgewählt aus der Gruppe bestehend aus Wasserstoff oder gegebenenfalls substituiertem C₁-C₆-Alkyl, C₃-C₁₂-Cycloalkyl,
oder ein Rest, ausgewählt aus der Gruppe bestehend aus gegebenenfalls substituiertem Pyridyl, Morpholinyl, Piperidinyl, Piperazinyl und Piperazinylcarbonyl, bedeutet.
Besonders bevorzugt sind Verbindungen der Formel (I), worin
R¹, R², R⁴, R⁵, n und L die angegebene Bedeutung aufweisen, und
R³ Wasserstoff, gegebenenfalls substituiertes C₁-C₆-Alkyl oder gegebenenfalls substituiertes und/oder verbrücktes C₃-C₁₂-Cycloalkyl,
bedeutet.
Insbesondere bevorzugt sind Verbindungen der Formel (I), worin
R¹, R², R³, R⁵, n und L die angegebene Bedeutung aufweisen, und
R⁴ ein Rest ausgewählt aus der Gruppe bestehend aus Wasserstoff, Hydroxy, Halogen, Methyl, Ethyl, Propinyloxy und Methoxy,
bedeutet.

Ein weiterer Gegenstand der Erfindung sind Verbindungen der Formel I zur Verwendung als Arzneimittel.

Erfindungsgemäß von besonderer Bedeutung sind Verbindungen der Formel I zur Verwendung als Arzneimittel mit antiproliferativer Wirkung.

Ein weiterer Gegenstand der Erfindung ist die Verwendung einer Verbindung der Formel I zur Herstellung eines Arzneimittels zur Behandlung und/oder Prävention von Krebs, Infektionen, Entzündungs- und Autoimmunerkrankungen. Ein weiterer Gegenstand der Erfindung ist eine Methode zur Behandlung und/oder Prävention von Krebs, Infektionen, Entzündungs- und Autoimmunerkrankungen, dadurch gekennzeichnet, dass man einem Patienten eine effektive Menge einer Verbindung der Formel I verabreicht.

Ein weiterer Gegenstand der Erfindung sind pharmazeutische Zubereitungen, enthaltend als Wirkstoff eine oder mehrere Verbindungen der allgemeinen Formel (I) oder deren physiologisch verträgliche Salze gegebenenfalls in Kombination mit üblichen Hilfs- und/oder Trägerstoffen.

Ein weiterer Gegenstand der Erfindung ist ein Verfahren zur Herstellung einer Verbindung der allgemeinen Formel (I), 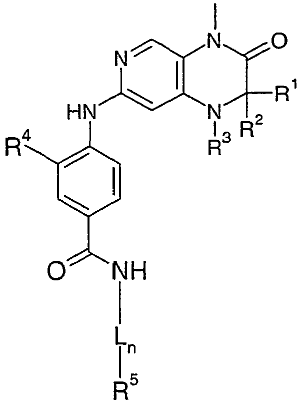 worin
R¹-R⁵ ,n und L die oben angegebene Bedeutung aufweisen,
dadurch gekennzeichnet, dass eine Verbindung der allgemeinen Formel (II) 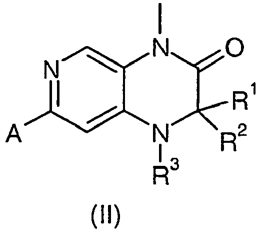 worin
R¹-R³ die oben 1 bis 4 angegebene Bedeutung aufweisen und A eine Abgangsgruppe ist,
mit einer gegebenenfalls substituierten Verbindung der allgemeinen Formel (III), 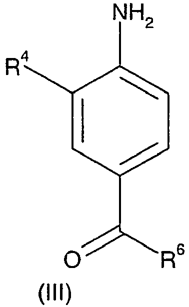 worin
R⁴ die oben 1 bis 5 angegebene Bedeutung aufweist und
R⁶ OH, -O-Methyl, -O-Ethyl bedeutet,
unter Erhalt eines Produkts der allgemeinen Formel (IV) umgesetzt wird, 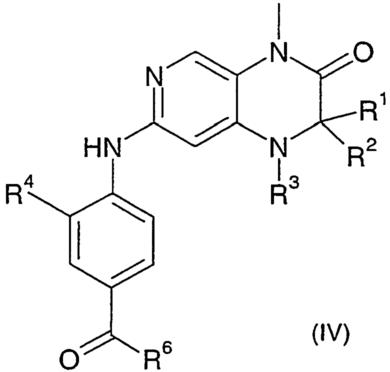 worin
R¹ bis R⁴ die oben angegebene Bedeutung aufweist und
R⁶ OH, -NH-Lₙ-R⁵, -O-Methyl oder -O-Ethyl bedeutet,
und gegebenenfalls anschliessend das erhaltene Produkt der allgemeinen Formel (IV) gegebenenfalls nach vorhergehender Hydrolyse der Estergruppe - COR⁶, mit einem Amin der allgemeinen Formel (V)

NH₂-Lₙ-R-⁵ (V)

worin
R⁵ die oben angegebene Bedeutung aufweist,
umgesetzt wird.

Die Erfindung betrifft darüber hinaus eine Verbindung der Formel (II), 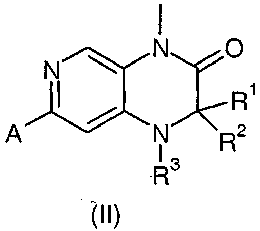 worin
R¹-R³ die oben angegebene Bedeutung aufweisen und A eine Abgangsgruppe ist.

Als Alkylgruppen sowie Alkylgruppen, welche Bestandteil anderer Reste sind, werden verzweigte und unverzweigte Alkylgruppen mit 1 bis 12 Kohlenstoffatomen bevorzugt 1 - 6, besonders bevorzugt 1-4 Kohlenstoffatomen bezeichnet, beispielsweise werden genannt: Methyl, Ethyl, Propyl, Butyl, Pentyl, Hexyl, Heptyl, Octyl, Nonyl, Decyl und Dodecyl. Sofern nicht anders genannt, sind von den vorstehend genannten Bezeichnungen Propyl, Butyl, Pentyl, Hexyl, Heptyl, Octyl, Nonyl, Decyl und Dodecyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.

In den vorstehend genannten Alkylgruppen können gegebenenfalls ein oder mehrere Wasserstoffatome durch andere Reste ersetzt sein. Beispielsweise können diese Alkylgruppen durch Fluor substituiert sein. Es können gegebenenfalls auch alle Wasserstoffatome der Alkylgruppe ersetzt sein.

Als Alkylbrücke werden, soweit nicht anders angegeben, verzweigte und unverzweigte Alkylgruppen mit 2 bis 5 Kohlenstoffatomen, beispielsweise Etyhlen, Propylen- , Isopropylen-, n-Butylen, iso-Butyl, sec. Butyl und tert.-Butyl etc. Brücken bezeichnet. Besonders bevorzugt sind Methylen, Etyhlen, Propylen- und Butylen-Brücken. In den genannten Alkylbrücken können gegebenenfalls 1 bis 2 C-Atome durch ein oder mehrere Heteroatome ausgewählt aus der Gruppe Sauerstoff, Stickstoff oder Schwefel, ersetzt sein.

Als Alkenylgruppen (auch soweit sie Bestandteil anderer Reste sind) werden verzweigte und unverzweigte Alkylengruppen mit 2 bis 12 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butenyl 1-Butenyl, 2-Butenyl, 3-Butenyl, 1-Methyl-1-Propenyl, 1-Methyl-2-Propenyl, 2-Methyl-1-Propenyl, 2-Methyl-2-Propenyl und 1-Ethyl-1-Ethenyl.

In den vorstehend genannten Alkenylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor substituiert sein. Es können gegebenenfalls auch alle Wasserstoffatome der Alkenylgruppe ersetzt sein.

Als Alkinylgruppen (auch soweit sie Bestandteil anderer Reste sind) werden verzweigte und unverzweigte Alkinylgruppen mit 2 bis 12 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkylgruppen Fluor substituiert sein. Es können gegebenenfalls auch alle Wasserstoffatome der Alkinylgruppe ersetzt sein.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere der nachfolgend genannten Substituenten tragen kann: OH, NO₂, CN, OMe, -OCHF₂, -OCF₃, -NH₂, Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, - COOH, -COO-C₁-C₄-Alkyl, vorzugsweise -COO-Methyl oder -COO-Ethyl, -CONH₂.

Als Heteroarylreste, in denen bis zu zwei C-Atome durch ein oder zwei Stickstoffatome ersetzt sind werden beispielsweise, Pyrrol, Pyrazol, Imidazol, Triazol, Pyridin, Pyrimidin, genannt, wobei jeder der vorstehend genannten Heteroarylringe gegebenenfalls ferner an einen Benzolring anneliert sein kann, vorzugsweise Benzimidazol, und wobei diese Heterocyclen ,soweit nicht anders beschrieben, beispielsweise einen oder mehrere der nachfolgend genannten Substituenten tragen können: F, Cl, Br, OH, OMe, Methyl, Ethyl, CN, CONH₂, NH₂, gegebenenfalls substituiertes Phenyl, gegebenenfalls substituiertes Heteroaryl, vorzugsweise gegebenenfalls substituiertes Pyridyl.

Als Cycloalkylreste werden Cycloalkylreste mit 3 - 12 Kohlenstoffatomen beispielsweise Cyclopropyl, Cyclobutyl, Cyclopentyl, Cyclohexyl, Cycloheptyl oder Cyclooctyl, vorzugsweise Cyclopropyl, Cyclopentyl oder Cyclohexyl bezeichnet, wobei jeder der vorstehend genannten Cycloalkylreste gegebenenfalls ferner einen oder mehrere Substituenten tragen kann, beispielsweise: OH, NO₂, CN, OMe, -OCHF₂, -OCF₃, -NH₂ oder Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -COO-Methyl oder -COO-Ethyl oder -CONH₂. Besonders bevorzugte Substituenten der Cycloalkylreste sind =O, OH, NH₂, Methyl oder F.

Als Cycloalkenylreste werden Cycloalkylreste mit 3 - 12 Kohlenstoffatomen, die mindestens eine Doppelbindung aufweisen, beispielsweise Cyclopropenyl, Cyclobutenyl, Cyclopentenyl, Cyclohexenyl oder Cycloheptenyl, vorzugsweise Cyclopropenyl, Cyclopententyl oder Cyclohexenyl bezeichnet, wobei jeder der vorstehend genannten Cycloalkenylreste gegebenenfalls ferner einen oder mehrere Substituenten tragen kann.

"=O" bedeutet ein über eine Doppelbindung verknüpftes Sauerstoffatom.

Als Polycycloalkylreste werden gegebenenfalls substituierte bi-, tri-, tetra- oder pentacyclische Cycloalkylreste, beispielsweise Pinan, 2.2.2-Octan, 2.2.1-Heptan oder Adamantan, bezeichnet. Als Polycycloalkenylreste werden gegebenenfalls verbrückte oder/und substituierte 8-gliedrige bi-, tri-, tetra- oder pentacyclische Cycloalkenyllreste, vorzugsweise Bicycloalkenyl- oder Tricycloalkenylreste, sofern sie mindestens eine Doppelbindung aufweisen, beispielsweise Norbornen, bezeichnet.

Als Spiroalkylreste werden gegebenenfalls substituierte spirocyclische C₅-C₁₂ Alkylreste bezeichnet.

Als Halogen wird im allgemeinen Fluor, Chlor, Brom oder Jod bezeichnet, vorzugsweise Fluor , Chlor oder Brom, besonders bevorzugt Chlor.

Als Abgangsgruppe A wird, gleich oder verschieden, eine Abgangsgruppe wie beispielsweise -O-Methyl, -SCN, Fluor, Chlor, Brom, Iod, Methansulfonyl, Trifluormethansulfonyl oder p-Toluolsulfonyl, vorzugsweise Chlor bezeichnet.

Die erfindungsgemäßen Verbindungen können in Form der einzelnen optischen Isomeren, Mischungen der einzelnen Enantiomeren, Diastereomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, sowie in Form ihrer Solvate oder Hydrate, vorzugsweise Mono- oder Dihydrate, vorliegen.

Der Substituent R¹ oder R² kann Wasserstoff oder ein Rest, ausgewählt aus der Gruppe bestehend aus gegebenenfalls substituiertem C₁-C₆-Alkyl, vorzugsweise Methyl, Ethyl oder Propyl, besonders bevorzugt Methyl oder Ethyl, C₂-C₆-Alkenyl, vorzugsweise Allyl, 1-Butenyl oder 2-Butenyl, besonders bevorzugt Allyl und C₂-C₆-Alkinyl, vorzugsweise Propinyl oder Butinyl, besonders bevorzugt Propinyl, bedeuten.

R¹ und R² können gemeinsam eine 2- bis 5-gliedrige Alkylbrücke, vorzugsweise eine Ethylen-, Propylen- oder Butylenbrücke, die 1 bis 2 Heteroatome enthalten kann, vorzugsweise Sauerstoff oder Stickstoff, bedeuten. Besonders bevorzugt sind Ethylen oder Propylen.

Der Substituent R³ kann Wasserstoff oder ein Rest, ausgewählt aus der Gruppe bestehend aus gegebenenfalls substituiertem C₁-C₁₂-Alkyl, vorzugsweise Propyl, Butyl, Pentyl, oder Hexyl besonders bevorzugt Propyl, Pentyl oder Hexyl, C₂-C₁₂-Alkenyl, vorzugsweise Butenyl, Pentenyl oder Hexenyl, besonders bevorzugt Pentenyl oder Hexenyl, C₂-C₁₂-Alkinyl, vorzugsweise Propinyl, Butinyl oder Pentinyl besonders bevorzugt Butinyl oder Pentinyl und C₆-C₁₄-Aryl, vorzugsweise Phenyl oder Naphthalinyl, oder ein Rest ausgewählt aus der Gruppe bestehend aus gegebenenfalls substituiertem und/oder verbrücktem C₃-C₁₂-Cycloalkyl, vorzugsweise Cyclopropyl, Cyclobutyl, Cyclopentyl oder Cyclohexyl, besonders bevorzugt Cyclopentyl oder Cyclohexyl, C₃-C₁₂-Cycloalkenyl, vorzugsweise Cyclopentenyl oder Cyclohexenyl, C₇-C₁₂-Polycycloalkyl, vorzugsweise 2.2.1-Heptanyl oder Adamantyl, C₇-C₁₂-Polycycloalkenyl, vorzugsweise Norbornenyl oder C₅-C₁₂-Spirocycloalkyl, vorzugsweise Spiro[4.4]nonyl oder Spiro[2.4]heptyl, bedeuten. Insbesondere bevorzugt bedeutet der Substituent R³ Isopentyl, Isopropyl Cyclohexyl oder Cyclopentyl.

Der Substituent R⁴ kann ein Rest ausgewählt aus der Gruppe bestehend aus Wasserstoff, Hydroxy und Halogen, vorzugsweise Fluor, Chlor oder Brom, besonders bevorzugt Fluor oder Chlor, oder
ein Rest ausgewählt aus der Gruppe bestehend aus gegebenenfalls substituiertem C₁-C₃-Alkyl, vorzugsweise Methyl, Ethyl, oder Propyl, besonders bevorzugt Methyl oder Ethyl, C₂-C₆-Alkenyl, vorzugsweise Allyl, 2-Butenyl oder 2-Pentenyl, besonders bevorzugt Allyl oder 2-Butenyl, C₂-C₆-Alkinyl, vorzugsweise Propinyl, 2-Butinyl oder 2-Pentinyl besonders bevorzugt Propinyl oder 2-Butinyl, C₁-C₅-Alkyloxy, vorzugsweise Methoxy, Ethoxy oder Propyloxy, besonders bevorzugt Methoxy oder Ethoxy, C₂-C₅-Alkenyloxy, vorzugsweise Allyloxy, 2-Butenyloxy oder 2-Pentenyloxy, besonders bevorzugt Allyloxy oder 2-Butenyloxy und C₂-C₅-Alkinyloxy, vorzugsweise 2-Propinyloxy, 2-Butinyloxy oder 2-Pentinyloxy, besonders bevorzugt 2-Propinyloxy oder 2-Butinyloxy bedeuten.
Insbesondere bevorzugt bedeutet der Substituent R⁴ Methoxy oder Ethyl.

L kann einen Linker ausgewählt aus der Gruppe bestehend aus gegebenenfalls substituiertem C₂-C₁₀-Alkyl, vorzugsweise Ethyl, Propyl, Butyl oder Pentyl, C₂-C₁₀-Alkenyl, C₆-C₁₄-Aryl, vorzugsweise Phenyl, -C₂-C₄-Alkyl-C₆-C₁₄-Aryl, -C₆-C₁₄-Aryl-C₁-C₄-Alkyl, vorzugsweise -Phenyl-methyl, gegebenenfalls verbrücktem C₃-C₁₂-Cycloalkyl, vorzugsweise Cyclohexyl, und Heteroaryl, das 1 oder 2 Stickstoffatome enthält, bedeuten.
n bedeutet 0 oder 1, vorzugsweise 1.

R⁵ kann ein Rest, ausgewählt aus der Gruppe bestehend aus Wasserstoff oder gegebenenfalls substituiertem C₁-C₆-Alkyl, vorzugsweise Methyl, Ethyl oder Benzyl, besonders bevorzugt Methyl oder Ethyl, oder
ein Rest, ausgewählt aus der Gruppe bestehend aus gegebenenfalls substituiertem Pyridyl, Morpholinyl, Piperidinyl, Piperazinyl, Piperazinylcarbonyl, Pyrrolidinyl, Tropenyl, Sulfoxomorpholinyl, Sulfonylmorpholinyl, Thiomorpholinyl und Azacycloheptyl, vorzugsweise Piperazinyl, Piperidinyl , Morpholinyl oder Pyrrolidinyl, besonders bevorzugt Piperazinyl oder Piperidinyl, bedeuten.

Der Substituent R⁶ kann OH, -O-Methyl, -O-Ethyl, vorzugsweise -O-Methyl oder -O-Ethyl bedeuten.

Alle in der Bedeutung für R¹ bis R⁶ genannten Reste können gegebenenfalls verzweigt und/oder substituiert sein.

Die Herstellung der erfindungsgemäßen Verbindungen kann nach den im folgenden beschriebenen Syntheseverfahren erfolgen, wobei die Substituenten der allgemeinen Formeln (X1) bis (X12) die zuvor genannten Bedeutungen haben. Diese Verfahren sind als Erläuterung der Erfindung zu verstehen ohne selbige auf deren Gegenstand zu beschränken.

Die Verbindungen der allgemeinen Formel (I) können nach folgendem Syntheseschema (I) hergestellt werden:

Die Verbindungen **X1**, **X2** und **X7** (R4 = H; R = Et (**X7a**) und R4 = OMe; R = Me (X7b)) sind kommerziell erhältlich; die Verbindung **X7c** mit R4 = OMe und R = Et kann nach literaturbekannten Vorschriften hergestellt werden: (a) Taran, F.; Renard, P. Y.; Bernard, H.; Mioskowski, C.; Frobert, Y.; et al.; J.Amer.Chem.Soc. 1998, 120, 3332-3339. (b) Ismail, Ibrahim A.; Sharp, Dale E.; Chedekel, Miles R.; J.Org.Chem. 1980, 45, 2243-2246.

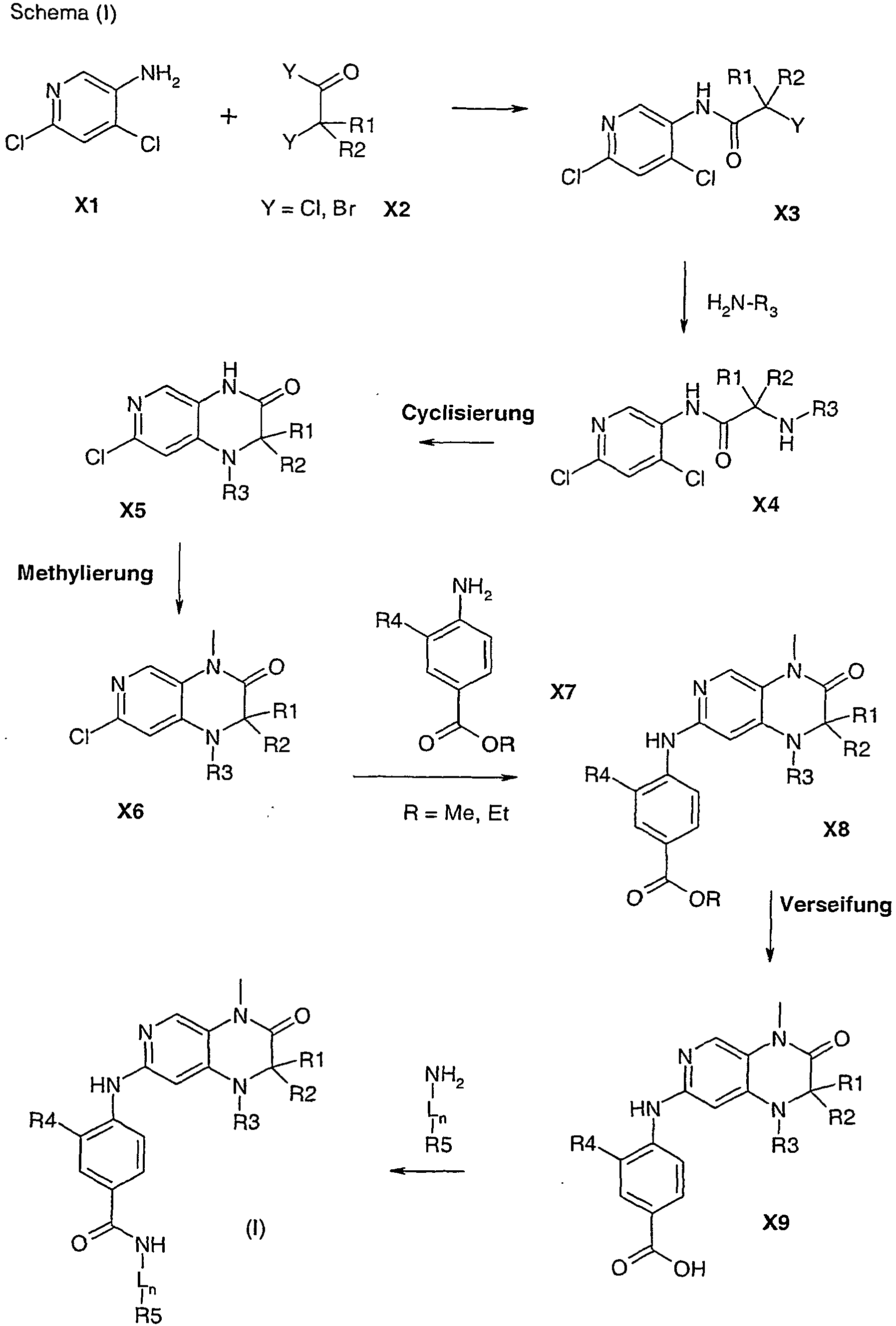

Alternativ kann folgender Syntheseweg, der besonders für die Herstellung enantiomerenreiner Verbindungen geeignet ist, angewendet werden (Schema (II)):
Verbindungen des Typs **X10** können nach literaturbekannten Vorschriften hergestellt werden: Lundquist, Joseph T.; Pelletier, IV and Jeffrey C, Org.Lett. 2001, 3, 781. Freudenberg; Kuhn; Bumann, Chem.Ber. 1930, 63, 2385.

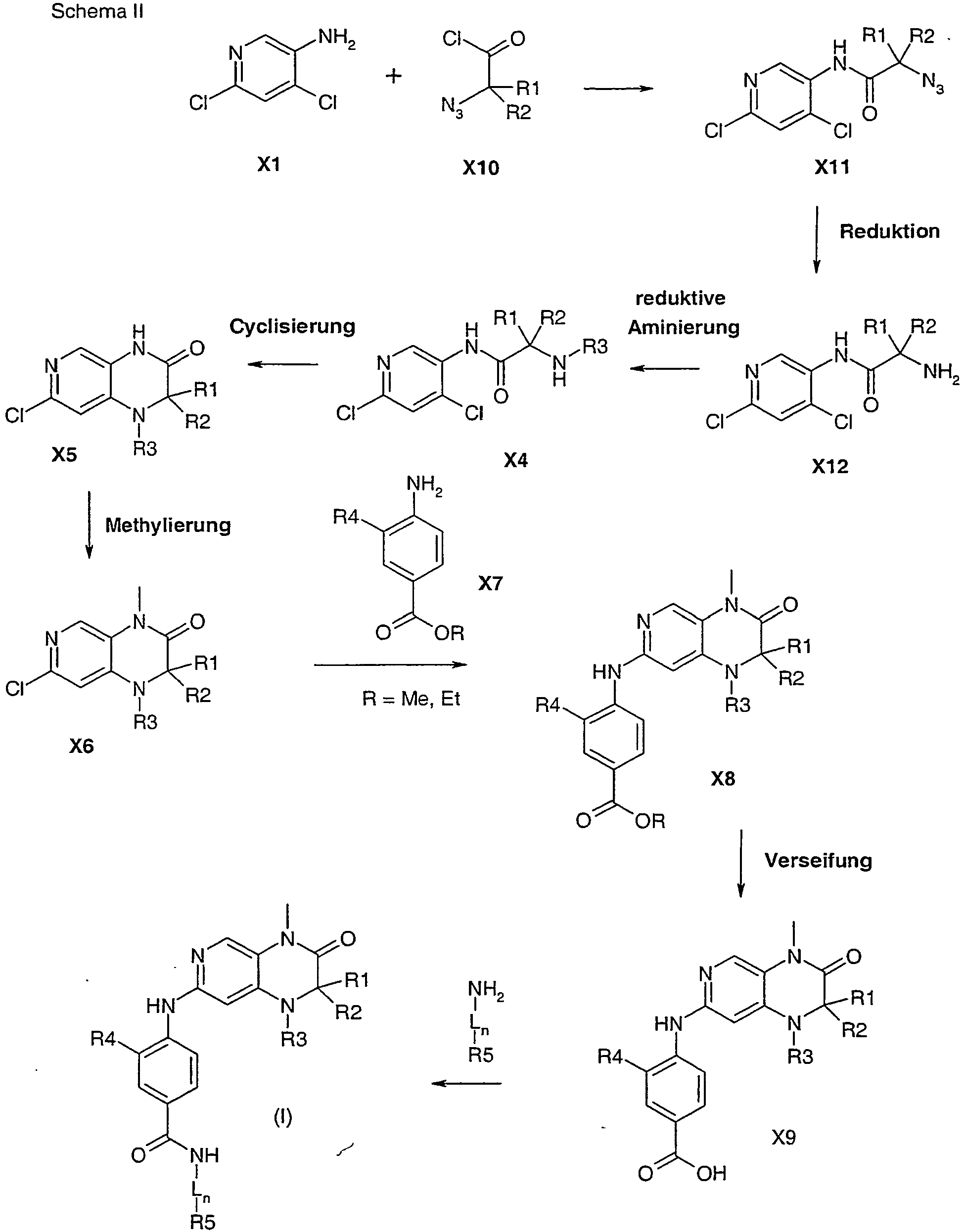

Die neuen Verbindungen der allgemeinen Formel (I) können in Analogie zu nachfolgenden Synthesebeispielen synthetisiert werden. Diese Beispiele sind allerdings nur als exemplarische Vorgehensweise zur weitergehenden Erläuterung der Erfindung zu verstehen, ohne selbige auf dessen Gegenstand zu beschränken.

Die Herstellung einiger zur Synthese der Beispiele eingesetzten Zwischenverbindungen wird ebenfalls im folgenden beschrieben.

### Zwischenverbindung 1:

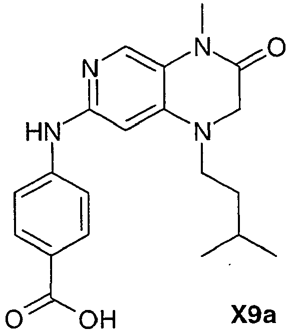

11.8 g 4,6-Dichlor-pyridin-3-yl-amin Dihydrochlorid wurden in 200 mL Dichlormethan und 200 mL Wasser vorgelegt und mit 30.0 g (215 mmol) Kaliumcarbonat versetzt. Die Reaktionsmischung wurde auf 0°C gekühlt und 11.3 g (100 mmol) Chloracetylchlorid zugetropft. Nach 30 Minuten wurde die organische Phase abgetrennt und eingeengt. Der Rückstand wurde mit Ether kristallisiert
Ausbeute: 8.6 g einer Verbindung **X3a** (farbloser Feststoff)

8.5 g der Verbindung **X3a** wurden in 85 mL Dimethylformamid vorgelegt und mit 13.9 g (100 mmol) Kaliumcarbonat und 8.2 mL (71 mmol) 3-Methylbutylamin versetzt. Die Reaktionsmischung wurde 2 Stunden bei 50°C gerührt, dann mit Wasser verdünnt. Die wässrigen Phase wurde zwei Mal mit Essigsäureethylester extrahiert. Die vereinten organische Phasen wurden über Na₂SO₄ getrocknet und eingeengt, der Rückstand in Methanol aufgenommen und mit etherischer Salzsäure-Lösung kristallisiert.
Ausbeute: 8.5 g einer Verbindung **X4a** (farbloser Feststoff)

8.4 g der Verbindung **X4a** wurden in 80 mL Dimethylformamid gelöst, mit 17.1 mL (100 mmol) N-Ethyldiisopropylamin versetzt und 2 Stunden auf 100°C erwärmt. Die Reaktionsmischung wurde mit Wasser verdünnt und die wässrige Phase zwei Mal mit Dichlormethan extrahiert. Die vereinten organische Phasen wurden über Na₂SO₄ getrocknet und eingeengt. Der Rückstand wurde aus Ether kristallisiert.
Ausbeute: 6.2 g einer Verbindung **X5a** (farbloser Feststoff)

6.2 g der Verbindung **X5a** wurden in 30 mL Dimethylacetamid vorgelegt und mit 2.4 mL (37 mmol) Methyliodid versetzt. Bei -10°C wurden portionsweise 1.3 g (30 mmol) Natriumhydrid als 60%ige Dispersion in Mineralöl zugegeben. Nach 30 Minuten wurde das Reaktionsgemisch auf Eiswasser gegossen. Der ausgefallene Niederschlag wurde abgesaugt, in Ether gelöst, über Na₂SO₄ getrocknet und eingeengt. Der Rückstand wurde aus Diisopropylether/Petrolether kristallisiert.
Ausbeute: 5.9 g einer Verbindung **X6a** (farbloser Feststoff)

Eine Suspension von 2.7 g der Verbindung **X6a** und 2.5 g (15 mmol) 4-Aminobenzoesäureethylester **X7a** in 20 mL Toluol wurde mit 0.6 g (1 mmol) 2,2'-Bis-(diphenylphosphino)-1,1'-binaphthyl, 0.46 g (0.5 mmol) Tris(dibenzylidenaceton)-dipalladium(0) und 6.5 g (20 mmol) Cäsiumcarbonat versetzt und 30 Stunden bei 100°C gerührt. Die Reaktionsmischung wurde mit 50 mL Essigsäureethylester und 50 mL Wasser verdünnt und der ausgefallene Feststoff abgesaugt. Die organische Phase des Filtrats wurde über Na₂SO₄ getrocknet und eingeengt. Der verbliebene Rückstand wurde mittels Säulenchromatographie (Laufmittel: Dichlormethan/Methanol 9:1) gereinigt. Ausbeute: 2.8 g einer Verbindung **X8a** (gelber Feststoff)
3 g der Verbindung **X8a** wurden in 100 mL Methanol gelöst, mit 15 mL 2N wässriger Natronlauge versetzt und 2 Stunden auf 60°C erwärmt. Die Reaktionsmischung wurde mit wässriger Salzsäure angesäuert und das Methanol im Vakuum entfernt. Der dabei entstandene Niederschlag wurde abgesaugt und mit Wasser und Aceton gewaschen.
Ausbeute: 2.4 g einer Verbindung **X9a** (farbloser Feststoff)

### Zwischenverbindung 2:

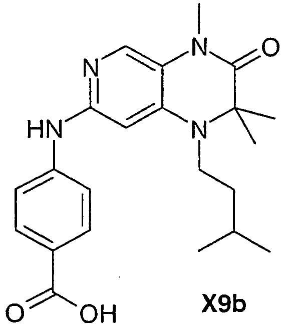

50 g (0.36 mol) Kaliumcarbonat wurden in 400 mL Wasser und 400 mL Ether gelöst und mit 20 g 4,6-Dichlor-pyridin-3-yl-amin Dihydrochlorid versetzt. Die Reaktionsmischung wurde auf 0°C gekühlt und eine Lösung von 18.9 mL (0.15 mol) 2-Brom-isobuttersäurebromid innerhalb von 2.5 Stunden zugetropft. Nach 2.5 Stunden wurden weitere 18.9 mL 2-Brom-isobuttersäurebromid, gelöst in 150 mL Ether sowie 20 g Kaliumcarbonat zugegeben und 1 Stunde gerührt. Die Reaktionsmischung wurde mit Essigsäureethylester verdünnt und mit Wasser gewaschen. Die organische Phase wurde abgetrennt, über Na₂SO₄ getrocknet und eingeengt. Der Rückstand wurde mittels Säulenchromatographie (Laufmittel: Cyclohexan/Essigsäureethylester 100:0 -> 90:10) gereinigt. Ausbeute: 20.6 g einer Verbindung **X3b** (farbloser Feststoff)

20 g der Verbindung **X3b** wurden in 80 mL Dimethylformamid vorgelegt und mit 16 g (120 mmol) Kaliumcarbonat und 31.6 mL (270 mmol) 3-Methylbutylamin versetzt. Die Reaktionsmischung wurde 1.5 Stunden bei 60°C gerührt, dann mit Wasser und Dichlormethan verdünnt. Die organische Phase wurde eingeengt, der Rückstand in Ether aufgenommen und das Produkt mit etherischer Salzsäure-Lösung kristallisiert. Der erhaltene Feststoff wurde in Kaliumhydrogencarbonat-Lösung aufgenommen, eingeengt und der Rückstand mittels Säulenchromatographie (Laufmittel: Cyclohexan/Essigsäureethylester 100:0 -> 25:75) gereinigt.
Ausbeute: 11.5 g einer Verbindung **X4b** (gelbes Öl)

11 g der Verbindung **X4b** wurden in 165 mL Dimethylformamid gelöst, mit 22 mL (130 mmol) N-Ethyldiisopropylamin versetzt und 72 Stunden auf 155°C erwärmt. Die Reaktionsmischung wurde eingeengt und der verbliebene Rückstand durch Zugabe von Wasser kristallisiert. Der Feststoff wurde mit Wasser und wenig Ether gewaschen.
Ausbeute: 6.2 g einer Verbindung **X5b** (hellgrauer Feststoff)

6.3 g der Verbindung **X5b** wurden in 30 mL Dimethylacetamid gelöst und mit 2.1 mL (33 mmol) Methyliodid versetzt. Bei -10°C wurden 1.2 g (27 mmol) Natriumhydrid als 60%ige Dispersion in Mineralöl portionsweise zugegeben. Die Reaktionsmischung wurde langsam auf Raumtemperatur erwärmt und dann auf Eiswasser gegossen. Der entstandene Niederschlag wurde abgesaugt und mit Wasser und Petrolether gewaschen.
Ausbeute: 6.0 g einer Verbindung **X6b** (farbloser Feststoff)

Eine Suspension von 2 g der Verbindung **X6b** und 1.7 g (10 mmol) 4-Aminobenzoesäureethylester **X7a** in 15 mL Toluol wurde mit 0.4 g (0.6 mmol) 2,2'-Bis-(diphenylphosphino)-1,1'-binaphthyl, 0.31 g (0.3 mmol) Tris(dibenzylidenaceton)-dipalladium(0) und 4.4 g (14 mmol) Cäsiumcarbonat versetzt und 35 Stunden bei 100°C gerührt. Die Reaktionsmischung wurde mit 100 mL Essigsäureethylester und 50 mL Wasser verdünnt und der entstandene Feststoff abfiltriert. Die organische Phase wurde über Na₂SO₄ getrocknet und eingeengt. Der Rückstand wurde mit tert-Butylmethylether versetzt und der erhaltene Feststoff abgesaugt.
Ausbeute: 1.2 g einer Verbindung **X8b** (brauner Feststoff)

1.7 g der Verbindung **X8b** wurden in 50 mL Methanol gelöst, mit einer Lösung von 1.6 g (40 mmol) Natriumhydroxid in 10 mL Wasser versetzt und 1.5 Stunden auf 60°C erwärmt. Die Reaktionsmischung wurde eingeengt und anschließend mit Wasser versetzt. Der dabei entstandene Niederschlag wurde abgesaugt und mit Ether und Petrolether gewaschen.
Ausbeute: 1.5 g einer Verbindung **X9b** (farbloser Feststoff)

### Zwischenverbindung 3:

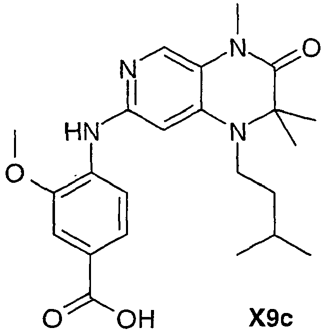

Eine Suspension von 2 g der Verbindung **X6b** und 1.8 g (10 mmol) 4-Amino-3-methoxybenzoesäuremethylester **X7b** in 15 mL Toluol wurde mit 0.4 g (0.7 mmol) 2,2'-Bis-(diphenylphosphino)-1,1'-binaphthyl, 0.3 g (0.3 mmol) Tris(dibenzylidenaceton)-dipalladium(0) und 4.4 g (14 mmol) Cäsiumcarbonat versetzt und 30 Stunden bei 100°C gerührt. Die Reaktionsmischung wurde mit 100 mL Essigsäureethylester und 50 mL Wasser verdünnt und der ausgefallene Feststoff abgesaugt. Die organische Phase des Filtrats wurde über Na₂SO₄ getrocknet und eingeengt. Der verbliebene Rückstand wurde mittels Säulenchromatographie (Laufmittel: Dichlormethan/Methanol 9:1) gereinigt. Das Produkt wurde durch Zugabe von Ether kristallisiert.
Ausbeute: 1.8 g einer Verbindung **X8c** (brauner Feststoff)

1.8 g der Verbindung **X8c** wurden in 50 mL Methanol gelöst, mit einer Lösung von 1.6 g (40 mmol) Natriumhydroxid in 10 mL Wasser versetzt und 1.5 Stunden auf 60°C erwärmt. Die Reaktionsmischung wurde eingeengt und anschließend mit Wasser versetzt. Der dabei entstandene Niederschlag wurde abgesaugt und mit Ether und Petrolether gewaschen.
Ausbeute: 1.7 g einer Verbindung **X9c** (farbloser Feststoff)

### Zwischenverbindung 4:

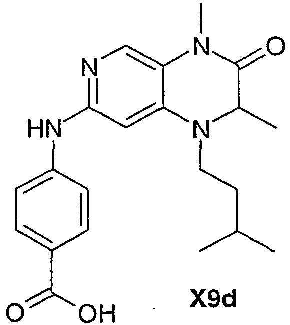

30.5 g 4,6-Dichlor-pyridin-3-yl-amin Dihydrochlorid wurden in 400 mL Ether und 300 mL Essigsäureethylester vorgelegt und bei 0°C mit einer Lösung von 75.9 g (0.55 mol) Kaliumcarbonat in 250 mL Wasser versetzt. Anschließend wurden 27.6 mL (0.26 mol) 2-Brompropionsäurebromid innerhalb von 30 Minuten zugetropft und die Reaktionsmischung innerhalb von 2 Stunden auf Raumtemperatur erwärmt. Entstandene Feststoffe wurden abfiltriert und das Filtrat mit Essigsäureethylester extrahiert. Die vereinten organischen Phasen wurden über Na₂SO₄ getrocknet, eingeengt und das erhaltene Produkt aus Ether kristallisiert.
Ausbeute: 32.5 g einer Verbindung **X3d** (farbloser Feststoff) 5.8 g der Verbindung **X3d** wurden in 50 mL Dimethylformamid vorgelegt und mit 5.2 g (38 mmol) Kaliumcarbonat und 6 g (69 mmol) 3-Methylbutylamin versetzt. Die Reaktionsmischung wurde 2 Stunden bei 80°C gerührt und anschließend mit Wasser verdünnt. Die wässrige Phase wurde zwei Mal mit Essigsäureethylester extrahiert. Die vereinten organische Phasen wurden mit Wasser gewaschen, über Na₂SO₄ getrocknet und eingeengt. Der Rückstand wurde durch Flashsäulenchromatographie (Laufmittel: Cyclohexan/Essigsäureethylester 75:25) gereinigt.
Ausbeute: 5.3 g einer Verbindung **X4d** (farbloser Feststoff)

5.3 g der Verbindung **X4d** wurden in 10 mL Dimethylformamid gelöst, mit 4.3 g (20 mmol) Tri-kaliumphosphat versetzt und 3 Stunden auf 125°C erwärmt. Die Reaktionsmischung wurde mit Wasser verdünnt und die wässrige Phase zwei Mal mit Essigsäureethylester extrahiert. Die vereinten organische Phasen wurden mit Wasser gewaschen, über Na₂SO₄ getrocknet und eingeengt. Der Rückstand wurde aus Petrolether kristallisiert.
Ausbeute: 2.2 g einer Verbindung **X5d** (farbloser Feststoff)

2.4 g der Verbindung **X5d** wurden in 15 mL Dimethylacetamid vorgelegt und mit 1 mL (16 mmol) Methyliodid versetzt. Bei -10°C wurden portionsweise 0.5 g (13 mmol) Natriumhydrid als 60%ige Dispersion in Mineralöl zugegeben. Nach 15 Minuten wurde das Reaktionsgemisch auf Eiswasser gegossen und zwei Mal mit Ether extrahiert. Die vereinten organischen Phasen wurden über Na₂SO₄ getrocknet und eingeengt. Der Rückstand wurde in Aceton aufgenommen und mit etherischer Salzsäure-Lösung kristallisiert.
Ausbeute: 2.4 g einer Verbindung **X6d** (farbloser Feststoff)

Eine Suspension von 1.2 g der Verbindung **X6d** und 1.7 g (10 mmol) 4-Aminobenzoesäureethylester **X7a** in 15 mL Toluol wurde mit 0.3 g (0.5 mmol) 2,2'-Bis-(diphenylphosphino)-1,1'-binaphthyl, 0.2 g (0.2 mmol) Tris(dibenzylidenaceton)-dipalladium(0) und 5 g (15 mmol) Cäsiumcarbonat versetzt und 18 Stunden unter Rückfluss gekocht. Die Reaktionsmischung wurde über Kieselgur abgesaugt und eingeengt. Der verbliebene Rückstand wurde mittels Säulenchromatographie (Laufmittel: Dichlormethan/Methanol 9:1) gereinigt. Das Produkt wurde in Aceton aufgenommen und durch etherische Salzsäure-Lösung als Hydrochlorid gefällt.
Ausbeute: 0.9 g einer Verbindung **X8d** (gelber Feststoff)

0.9 g der Verbindung **X8d** wurden in 15 mL Wasser suspendiert, mit 15 mL halbkonz. Salzsäure versetzt und 2 Stunden unter Rückfluss gekocht. Nach dem Abkühlen wurde der entstandene Niederschlag abgesaugt und mit Wasser, Aceton und Ether gewaschen.
Ausbeute: 0.8 g einer Verbindung **X9d** (farbloser Feststoff)

### Zwischenverbindung 5:

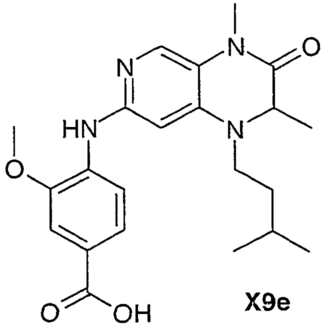

Eine Suspension von 1.5 g der Verbindung **X6d** und 2.7 g (15 mmol) 4-Amino-3-methoxybenzoesäuremethylester **X7b** in 30 mL Toluol wurde mit 1.3 g (2 mmol) 2,2'-Bis-(diphenylphosphino)-1,1'-binaphthyl, 0.9 g (1 mmol) Tris(dibenzylidenaceton)-dipalladium(0) und 10 g (30 mmol) Cäsiumcarbonat versetzt und 18 Stunden unter Rückfluss gekocht. Nach dem Abkühlen wurde der entstandene Feststoff abgesaugt und das Filtrat eingeengt. Der verbliebene Rückstand wurde mittels Säulenchromatographie (Laufmittel: Dichlormethan/Methanol 9:1) gereinigt.
Ausbeute: 0.3 g einer Verbindung **X8e** (brauner Feststoff)

0.3 g der Verbindung **X8e** wurden in 10 mL 2N wässriger Salzsäure suspendiert und 2 Stunden unter Rückfluss gekocht. Nach dem Abkühlen wurde der entstandene Niederschlag abgesaugt und mit Wasser, Aceton und Ether gewaschen.
Ausbeute: 0.2 g einer Verbindung **X9e** (farbloser Feststoff)

### Zwischenverbindung 6:

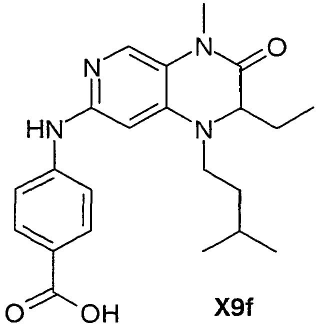

10 g 4,6-Dichlor-pyridin-3-yl-amin wurden in 200 mL Ether gelöst und mit einer Lösung von 20 g (0.14 mol) Kaliumcarbonat versetzt. Bei 0°C wurden 15 mL (0.11 mol) 2-Brombutyrylbromid innerhalb von zwei Stunden zugetropft, wobei ein Feststoff entstand. Die Reaktionsmischung wurde mit 200 mL Essigsäureethylester verdünnt, die organische Phase über Na₂SO₄ getrocknet und eingeengt. Der erhaltene Feststoff wurde mit Ether gewaschen.
Ausbeute: 14.5 g einer Verbindung **X3f** (farbloser Feststoff)

Eine Mischung aus 14.2 g der Verbindung **X3f**, 20 g (0.14 mol) Kaliumcarbonat und 4.4 g (50 mmol)) 3-Methylbutylamin in 40 mL Dimethylformamid wurde vier Stunden bei 120°C gerührt und anschließend eingeengt. Das verbliebene Öl wurde mit Wasser versetzt und zwei Mal mit Essigsäureethylester extrahiert. Die vereinten organischen Phasen wurden mit Wasser gewaschen, über Na₂SO₄ getrocknet und eingeengt. Der Rückstand wurde in Essigsäureethylester aufgenommen und das Produkt mit etherischer Salzsäure kristallisiert. Der erhaltene Feststoff wurde mit Essigsäureethylester und Ether gewaschen.
Ausbeute: 10.2 g einer Verbindung **X4f** (farbloser Feststoff)

Eine Mischung aus 11.7 g der Verbindung **X4f** und 10.3 g (80 mmol) N-Ethyldiisopropylamin in 50 mL Dimethylformamid wurde 9 Stunden unter Rückfluss gekocht. Die Reaktionsmischung wurde eingeengt, mit wässriger Kaliumcarbonat-Lösung versetzt und zwei Mal mit Essigsäureethylester extrahiert. Die vereinten organischen Phasen wurden über Na₂SO₄ getrocknet und eingeengt. Der Rückstand wurde aus Ether kristallisiert.
Ausbeute: 8.3 g einer Verbindung **X5f** (farbloser Feststoff)

8.2 g der Verbindung **X5f** wurden in 100 mL Dimethylformamid vorgelegt, mit 2.8 mL (45 mmol) Methyliodid versetzt und bei -10°C 1.8 g (45 mmol) Natriumhydrid als 60%ige Dispersion in Mineralöl portionsweise zugegeben. Die Reaktionsmischung wurde 30 Minuten bei 0°C gerührt, auf 400 mL Eiswasser gegossen und die wässrige Phase zwei Mal mit Essigsäureethylester extrahiert. Die vereinten organischen Phasen wurden über Na₂SO₄ getrocknet und eingeengt. Der Rückstand wurde in Aceton/Ether aufgenommen und das Produkt mit etherischer Salzsäure-Lösung kristallisiert.
Ausbeute: 9.1 g einer Verbindung **X6f** (farbloser Feststoff)

Eine Suspension von 1.3 g der Verbindung **X6f** und 1.7 g (10 mmol) 4-Aminobenzoesäureethylester **X7a** in 15 mL Toluol wurde mit 0.4 g (0.6 mmol) 2,2'-Bis-(diphenylphosphino)-1,1'-binaphthyl, 2.5 g (2.7 mmol) Tris(dibenzylidenaceton)-dipalladium(0) und 1.5 g (5 mmol) Cäsiumcarbonat versetzt und 24 Stunden bei 100°C gerührt. Die Reaktionsmischung wurde mit 50 mL Essigsäureethylester versetzt und der entstandene Niederschlag abfiltriert. Das Filtrat wurde eingeengt und mittels Säulenchromatographie (Laufmittel: Dichlormethan/Methanol 9:1) gereinigt. Das Produkt wurde in Aceton aufgenommen und durch etherische Salzsäure-Lösung als Hydrochlorid gefällt.
Ausbeute: 1.6 g einer Verbindung **X8f** (gelber Feststoff)

1.6 g der Verbindung **X8f** wurden in 60 mL 1 N wässriger Salzsäure suspendiert und 24 Stunden unter Rückfluss gekocht. Nach dem Abkühlen wurde der entstandene Niederschlag abgesaugt und mit Wasser und Aceton gewaschen.
Ausbeute: 1.4 g einer Verbindung **X9f** (farbloser Feststoff)

### Zwischenverbindung 7:

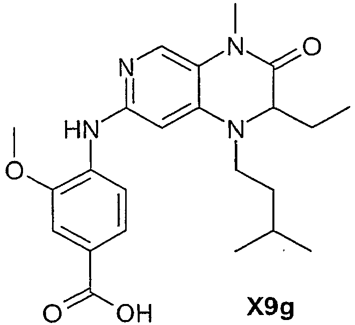

Eine Suspension von 3.4 g der Verbindung **X6f** und 4.4 g (24 mmol) 4-Amino-3-methoxybenzoesäuremethylester **X7b** in 60 mL Toluol wurde mit 0.9 g (1.5 mmol) 2,2'-Bis-(diphenylphosphino)-1,1'-binaphthyl, 0.9 g (1 mmol) Tris(dibenzylidenaceton)-dipalladium(0) und 10 g (30 mmol) Cäsiumcarbonat versetzt und 24 Stunden auf 100°C erwärmt. Nach dem Abkühlen wurde der entstandene Feststoff abgesaugt und das Filtrat eingeengt. Der verbliebene Rückstand wurde mittels Säulenchromatographie (Laufmittel: Cyclohexan/Essigsäureethylester 3:1) gereinigt.
Ausbeute: 4.6 g einer Verbindung **X8g** (brauner Feststoff)

4.4 g der Verbindung **X8g** wurden in 60 mL 2N wässriger Salzsäure suspendiert und 18 Stunden unter Rückfluss gekocht. Nach dem Abkühlen wurde der entstandene Niederschlag abgesaugt und mit Aceton und Ether gewaschen.
Ausbeute: 3.9 g einer Verbindung **X9g** (farbloser Feststoff)

### Zwischenverbindung 8:

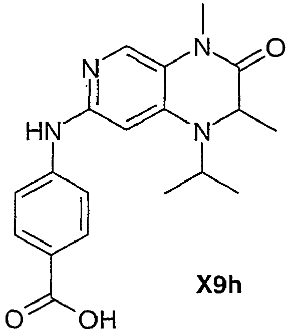

20.7 g der Verbindung **X3d** wurden in 150 mL Acetonitril vorgelegt, mit 59.6 mL (0.7 mol) Isopropylamin versetzt und 24 Stunden bei Raumtemperatur gerührt. Die Reaktionsmischung wurde eingeengt, in Wasser aufgenommen und mit Dichlormethan extrahiert. Die vereinten organischen Phasen wurden über Na₂SO₄ getrocknet und eingeengt. Das erhaltene Öl wurde in Ether und wenig Essigsäureethylester aufgenommen und das Produkt durch Zugabe von etherischer Salzsäure-Lösung kristallisiert.
Ausbeute: 20.5 g einer Verbindung **X4h** (farbloser Feststoff)

Eine Mischung von 9.1 g der Verbindung **X4h** und 15 g (0.11 mol) N-Ethyldiisopropylamin in 50 mL Dimethylformamid wurde 7 Tage unter Rückfluss erwärmt und anschließend eingeengt. Der Rückstand wurde in wässriger Kaliumcarbonat-Lösung aufgenommen und mit Essigsäureethylester extrahiert. Die vereinten organischen Phasen wurden über Na₂SO₄ getrocknet, eingeengt und der Rückstand mit Ether kristallisiert.
Ausbeute: 5.5 g einer Verbindung **X5h** (leicht gelber Feststoff)

18.2 g der Verbindung **X5h** wurden in 250 mL Dimethylformamid vorgelegt und mit 8.2 mL (0.13 mol) Methyliodid versetzt. Die Mischung wurde auf -5°C gekühlt und 4.0 g (0.10 mol) Natriumhydrid als 60%ige Dispersion in Mineralöl portionsweise zugegeben. Die Reaktionsmischung wurde zwei Stunden bei 0°C gerührt und dann auf 800 mL Eiswasser gegossen. Der entstandene Niederschlag wurde abfiltriert und mit Wasser und Petrolether gewaschen.
Ausbeute: 15.9 g einer Verbindung **X6h** (leicht gelber Feststoff)

Eine Suspension von 1 g der Verbindung **X6h** und 1.4 g (10 mmol) 4-Aminobenzoesäureethylester **X7a** in 60 mL Toluol wurde mit 0.2 g (0.3 mmol) 2,2'-Bis-(diphenylphosphino)-1,1'-binaphthyl, 0.3 g (0.3 mmol) Tris(dibenzylidenaceton)-dipalladium(0) und 3.3 g (10 mmol) Cäsiumcarbonat versetzt und 65 Stunden bei 100°C gerührt. Die Reaktionsmischung wurde über Cellulose filtriert, eingeengt und der Rückstand mittels Säulenchromatographie (Laufmittel: Essigsäureethylester/Petrolether 2:1) gereinigt.
Ausbeute: 1.2 g einer Verbindung **X8h** (gelber Schaum)

1 g der Verbindung **X8h** wurden in 15 mL Wasser suspendiert, mit 15 mL halbkonz. Salzsäure versetzt und 2 Stunden unter Rückfluss gekocht. Nach dem Abkühlen wurde der entstandene Niederschlag abgesaugt und mit Wasser, Aceton und Ether gewaschen.
Ausbeute: 0.9 g einer Verbindung **X9h** (farbloser Feststoff)

### Zwischenverbindung 9:

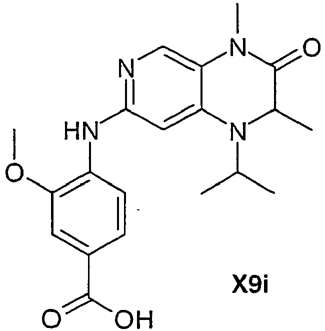

Eine Suspension von 2.9 g der Verbindung **X6h** und 4.4 g (23 mmol) 4-Amino-3-methoxybenzoesäureethylester **X7c** in 120 mL Toluol wurde mit 0.8 g (1.3 mmol) 2,2'-Bis-(diphenylphosphino)-1,1'-binaphthyl, 1 g (1.1 mmol) Tris(dibenzylidenaceton)-dipalladium(0) und 10 g (30 mmol) Cäsiumcarbonat versetzt und 72 Stunden auf 100°C erwärmt. Nach dem Abkühlen wurde das Reaktionsgemisch über Cellulose filtriert und das Filtrat eingeengt. Der verbliebene Rückstand wurde mittels Säulenchromatographie (Laufmittel: Petrolether/Essigsäureethylester 1:2) gereinigt.
Ausbeute: 3.6 g einer Verbindung **X8i** (leicht brauner Feststoff)

3.6 g der Verbindung **X8i** wurden in 20 mL Wasser suspendiert, mit 15 mL halbkonz. Salzsäure versetzt und 2 Stunden unter Rückfluss gekocht. Nach dem Abkühlen wurde der entstandene Niederschlag abgesaugt und mit Wasser, Aceton und Ether gewaschen.
Ausbeute: 2.95 g einer Verbindung **X9i** (farbloser Feststoff)

### Zwischenverbindung 10:

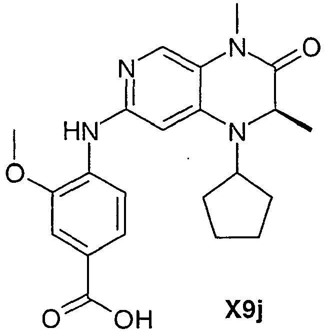

10 g (154 mmol) Natriumazid wurden in 45 mL Wasser gelöst, bei 0°C mit 75 mL Dichlormethan und 9.3 mL (55 mmol) Trifluormethansulfonsäureanhydrid versetzt. Die Reaktionsmischung wurde zwei Stunden gerührt und dann zwei Mal mit je 40 mL Dichlormethan extrahiert. Die vereinten organischen Phasen wurden mit gesättigter wässriger Natriumhydrogencabonat-Lösung gewaschen und über Na₂SO₄ getrocknet. Die so erhaltene Lösung wurde zu einer Mischung aus 2.5 g (28 mmol) D-Alanin, 5.9 g (42 mmol) Kaliumcarbonat und 70 mg (0.3 mmol) Kupfer-(II)sulfat-Pentahydrat in 90 mL Wasser und 180 mL Methanol gegeben. Es wurde 12 Stunden bei Raumtemperatur gerührt und dann die organischen Lösungsmittel im Vakuum entfernt. Der Rückstand wurde mit Wasser verdünnt und ein pH-Wert von 6.2 eingestellt. Anschließend wurde mit Essigsäureethylester extrahiert. Die wässrige Phase wurde nun auf pH 2 eingestellt und erneut mit Essigsäureethylester extrahiert. Diese organischen Phasen wurden über Na₂SO₄ getrocknet und eingeengt.
Ausbeute: 4.0 g einer Verbindung **X13a** (leicht gelbes Öl)

3.3 g der Verbindung **X13a** wurden in 30 mL Dichlormethan gelöst, mit 5 mL (68 mmol) Thionylchlorid versetzt und zwei Stunden bei 50°C gerührt. Die Mischung wurde einrotiert und der Rückstand mit einer Lösung von 6.6 g (28 mmol) 4,6-Dichlor-pyridin-3-yl-amin Dihydrochlorid und 10 mL (124 mmol) Pyridin in 10 mL Dichlormethan versetzt. Nach 12 Stunden wurde mit Wasser versetzt und mit Dichlormethan extrahiert. Die vereinten organischen Phasen wurden über Na₂SO₄ getrocknet, eingeengt und mittels Säulenchromatographie (Laufmittel: Dichlormethan/Methanol 100:5) gereinigt.
Ausbeute: 4.9 g einer Verbindung **X11a** (leicht brauner Feststoff)

In einer Argon-Atmosphäre wurden 7.1 g der Verbindung **X11a** in 150 mL THF vorgelegt und tropfenweise mit 35 mL einer 1 M Lösung von Trimethylphosphin in THF versetzt. Es wurde über Nacht gerührt, dann mit Wasser versetzt und eingeengt. Der Rückstand wurde in Wasser aufgenommen und mit Dichlormethan extrahiert. Die vereinten organischen Phasen wurden über Na₂SO₄ getrocknet und eingeengt. Das erhaltene gelbe Öl wurde in Aceton/Ether aufgenommen und das Produkt durch Zugabe von etherischer Salzsäure-Lösung kristallisiert.
Ausbeute: 5.4 g einer Verbindung **X12a** (farbloser Feststoff)

6.3 g der Verbindung **X12a** wurden in 250 mL Dichlormethan vorgelegt und mit 2.2 g (26 mmol) Cyclopentanon, 14 g (66 mmol) Natriumtriacetoxyborhydrid und 3.3 g (40 mmol) Natriumacetat versetzt. Es wurde 12 Stunden gerührt und anschließend mit wässriger Natriumhydrogencabonat-Lösung versetzt. Die organische Phase wurde über Na₂SO₄ getrocknet und eingeengt Das erhaltene Öl wurde mittels Säulenchromatographie (Laufmittel Dichlormethan/Methanol 100:2) gereinigt.
Ausbeute: 5.5 einer Verbindung **X4j** (leicht gelbes Öl)

Eine Mischung von 1 g der Verbindung **X4j** und 3.5 mL (20 mmol) N-Ethyldiisopropylamin in 5 mL Dimethylformamid wurde 34 Stunden auf 150°C erwärmt. Die Reaktionsmischung wurde mit Wasser versetzt und mit Dichlormethan extrahiert. Die vereinten organischen Phasen wurden über Na₂SO₄ getrocknet und eingeengt.
Ausbeute: 0.7 g einer Verbindung **X5j** (beiger Feststoff)

4.1 g der Verbindung **X5j** wurden in 10 mL Dimethylformamid vorgelegt und mit 1 mL (17 mmol) Methyliodid versetzt. Bei -5°C wurden nun 1.2 g (30 mmol) Natriumhydrid als 60%ige Dispersion in Mineralöl portionsweise zugegeben. Die Reaktionsmischung wurde zwei Stunden bei 0°C gerührt, mit Wasser versetzt und mit Dichlormethan extrahiert. Die vereinten organische Phasen wurden über Na₂SO₄ getrocknet und eingeengt.
Ausbeute: 4.0 g einer Verbindung **X6j** (oranges Öl)

Eine Suspension von 4 g der Verbindung **X6j** und 2.8 g (15 mmol) 4-Amino-3-methoxybenzoesäuremethylester **X7b** in 70 mL Toluol wurde mit 0.6 g (0.9 mmol) 2,2'-Bis-(diphenylphosphino)-1,1'-binaphthyl, 0.9 g (0.9 mmol) Tris(dibenzylidenaceton)-dipalladium(0) und 12 g (37 mmol) Cäsiumcarbonat versetzt und 50 Stunden unter Rückfluss gekocht. Nach dem Abkühlen wurde das Reaktionsgemisch über Cellulose filtriert und das Filtrat eingeengt. Der verbliebene Rückstand wurde in Essigsäureethylester aufgenommen, mit Wasser gewaschen, über Na₂SO₄ getrocknet, über Aktivkohle filtriert und eingeengt. Das Produkt wurde durch Säulenchromatographie (Laufmittel: Dichlormethan/Methanol 95:5) gereinigt.
Ausbeute: 3.1 g einer Verbindung **X8j** (rotes Öl)

3.1 g der Verbindung **X8j** wurden in 30 mL Wasser suspendiert, mit 15 mL konz. Salzsäure versetzt und 10 Stunden unter Rückfluss gekocht. Nach dem Abkühlen wurde der entstandene Niederschlag abgesaugt und mit Wasser, Aceton und Ether gewaschen.
Ausbeute: 2.2 g einer Verbindung **X9j** (farbloser Feststoff)

### Synthese ausgewählter Beispiele aus Tabelle 1:

### Beispiel 2:

0.1 g der Verbindung **X9d**, 0.08 g TBTU und 1 mL DIPEA wurden in 2 mL Dimethylformamid gelöst und 10 Minuten bei 25°C gerührt. Dann wurden 0.05 g 3-Picolylamin zugegeben und für weitere 15 Minuten bei 70°C gerührt. Die Reaktionsmischung wurde eingeengt, mit 10 mL Essigsäureethylester versetzt und mit Wasser gewaschen. Die wässrige Phase wurde mit Essigsäureethylester extrahiert. Die vereinten organischen Phasen wurden mit Wasser gewaschen, über Na₂SO₄ getrocknet und eingeengt. Der Rückstand wurde aus Essigsäureethylester kristallisiert.
Ausbeute: 0.067 g (farbloser Feststoff) Smp. 167-168°C

### Beispiel 5:

0.06 g der Verbindung **X9e**, 0.055 g TBTU und 0.5 g DIPEA wurden in 2 mL Dimethylformamid gelöst und 10 Minuten bei 25°C gerührt. Dann wurden 0.05 g 4-Picolylamin zugegeben und weitere 30 Minuten bei 25°C gerührt. Die Reaktionsmischung wurde eingeengt und mit 20 mL Essigsäureethylester und 10 mL Wasser versetzt. Die wässrige Phase wurde mit Essigsäureethylester extrahiert. Die vereinten organischen Phasen wurden mit Wasser gewaschen, über Na₂SO₄ getrocknet und eingeengt. Der Rückstand wurde säulenchromatographisch (Laufmittel: Dichlormethan/Essigsäureethylester/Methanol 70:20:10) gereinigt.
Ausbeute: 0.043 g (farbloser Feststoff)

### Beispiel 6:

0.23 g der Verbindung **X9g**, 0.18 g TBTU und 0.29 mL DIPEA wurden in 5 mL Dimethylformamid gelöst und 10 Minuten bei 25°C gerührt. Dann wurden 0.07 g 3-Picolylamin zugegeben und weitere 15 Minuten bei 25°C gerührt. Die Reaktionsmischung wurde eingeengt und mit 20 mL Essigsäureethylester und 10 mL Wasser versetzt. Die wässrige Phase wurde mit Essigsäureethylester extrahiert. Die vereinten organischen Phasen wurden mit Wasser gewaschen, über Na₂SO₄ getrocknet und eingeengt. Der Rückstand wurde säulenchromatographisch (Laufmittel: Dichlormethan/Essigsäureethylester/Methanol 70:20:10) gereinigt und als Hydrochlorid aus Essigsäureethylester gefällt.
Ausbeute: 0.16 g (farbloser Feststoff) Smp. 104-112°C

### Beispiel 8:

0.23 g der Verbindung **X9g**, 0.18 g TBTU und 0.30 mL DIPEA wurden in 5 mL Dimethylformamid gelöst und 10 Minuten bei 25°C gerührt. Dann wurden 0.06 g Cyclopropylamin zugegeben und weitere 15 Minuten bei 25°C gerührt. Die Reaktionsmischung wurde eingeengt und mit 20 mL Essigsäureethylester und 10 mL wässriger Kaliumcarbonat Lösung versetzt. Die wässrige Phase wurde mit Essigsäureethylester extrahiert. Die vereinten organischen Phasen wurden mit Wasser gewaschen, über Na₂SO₄ getrocknet und eingeengt. Der Rückstand wurde säulenchromatographisch (Laufmittel: Dichlormethan/Essigsäureethylester/Methanol 70:20:10) gereinigt und aus Essigsäureethylester kristallisiert. Der erhaltene Feststoff wurde mit Ether gewaschen.
Ausbeute: 0.12 g (farbloser Feststoff) Smp. 201-203°C

### Beispiel 13:

0.15 g der Verbindung **X9f**, 0.15 g TBTU und 0.29 mL DIPEA wurden in 5 mL Dichlormethan gelöst und 30 Minuten bei 25°C gerührt. Dann wurden 0.10 g Cyclobutylamin zugegeben und über Nacht bei 25°C gerührt. Die Reaktionsmischung wurde eingeengt und der Rückstand mit 20 mL Wasser versetzt. Der entstandene Niederschlag wurde abgesaugt, in Dichlormethan aufgenommen und diese Lösung über Na₂SO₄ getrocknet und eingeengt. Das Produkt wurde aus Aceton durch Zugabe von etherischer Salzsäure-Lösung kristallisiert.
Ausbeute: 0.13 g (farbloser Feststoff) Smp. 238-239°C

### Beispiel 18:

Eine Mischung von 0.3 g der Verbindung **X9a** und 0.3 g Thionylchlorid in 30 mL Dichlormethan wurde 24 Stunden unter Rückfluss gekocht. Die Reaktionsmischung wurde eingeengt, in 10 mL Dichlormethan aufgenommen, mit 0.5 g 3-Aminopyridin versetzt und 30 Minuten bei 25°C gerührt. Nach dem Einengen wurde der Rückstand mit Wasser verdünnt und der entstandene Niederschlag abgesaugt und mit Wasser gewaschen. Der Feststoff wurde in Dichlormethan gelöst, über Na₂SO₄ getrocknet und eingeengt. Das Produkt wurde durch Zugabe von Aceton kristallisiert.
Ausbeute: 0.09 g (farbloser Feststoff) Smp. 231-232°C

### Beispiel 22:

0.1 g der Verbindung **X9b**, 0.09 g TBTU und 0.30 mL DIPEA wurden in 5 mL Dichlormethan gelöst und 20 Minuten bei 25°C gerührt. Dann wurden 0.04 g 1-Methyl-piperidin-4-ylamin zugegeben und weitere 4 Stunden bei 25°C gerührt. Die Lösung wurde mit 15 mL Dichlormethan verdünnt und mit Wasser gewaschen. Die organische Phase wurde eingeengt und der Rückstand durch Zugabe von Ether und Essigsäureethylester gefällt. Der erhaltene Feststoff wurde mit Methanol/Ether verrührt und abgesaugt.
Ausbeute: 0.024 g (farbloser Feststoff)

### Beispiel 25:

0.1 g der Verbindung **X9c**, 0.09 g TBTU und 0.30 mL DIPEA wurden in 2 mL Dimethylformamid gelöst und 20 Minuten bei 25°C gerührt. Dann wurden 0.04 g 4-Aminopyridin zugegeben und weitere 1.5 Stunden bei 100°C gerührt. Die Lösung wurde mit 15 mL Dichlormethan verdünnt und mit Wasser gewaschen. Die organische Phase wurde eingeengt und der Rückstand durch Säulenchromatographie (Laufmittel: Dichlormethan -> Dichlormethan/Methanol 90:10) gereinigt. Das Produkt wurde durch Zugabe von Petrolether, Ether und Essigsäureethylester gefällt.
Ausbeute: 0.01 g (farbloser Feststoff) Smp. 117°C

### Beispiel 44

0.15 g der Verbindung **X9i**, 0.15 g TBTU und 0.10 mL DIPEA wurden in 1 mL Dichlormethan gelöst und 30 Minuten bei 25°C gerührt. Dann wurden 0.07 g 8-Methyl-8-aza-bicyclo[3.2.1]oct-3-ylamin zugegeben und über Nacht bei 25°C gerührt. Die Reaktionsmischung wurde mit wässriger Kaliumcarbonat-Lösung gewaschen und die organische Phase eingeengt. Der Rückstand wurde durch Zugabe von Ether kristallisiert.
Ausbeute: 0.16 g (farbloser Feststoff) Smp. >200°C

### Beispiel 45:

0.15 g der Verbindung **X9i**, 0.14 g TBTU und 0.11 mL DIPEA wurden in 1 mL Dichlormethan gelöst und 30 Minuten bei 25°C gerührt. Dann wurden 0.15 mL Ammoniak als 7 N Lösung in Methanol zugegeben und über Nacht bei 25°C gerührt. Die Reaktionsmischung wurde abfiltriert und das Filtrat mit wässriger Kaliumcarbonat-Lösung gewaschen. Die organische Phase wurde eingeengt und der Rückstand durch Zugabe von Ether kristallisiert.
Ausbeute: 0.13 g (farbloser Feststoff) Smp. >200°C

### Beispiel 46:

0.1 g der Verbindung **X9h**, 0.11 g TBTU und 0.07 mL DIPEA wurden in 1 mL Dichlormethan gelöst und 30 Minuten bei 25°C gerührt. Dann wurden 0.03 g 3-Aminopyridin zugegeben und über Nacht bei 25°C gerührt. Die Reaktionsmischung wurde mit wässriger Kaliumcarbonat-Lösung gewaschen und die organische Phase eingeengt. Der Rückstand wurde säulenchromatographisch (Laufmittel: Dichlormethan/Methanol 100:5 bis 100:7) gereinigt und das Produkt durch Zugabe von Ether kristallisiert.
Ausbeute: 0.04 g (gelblicher Feststoff) Smp. >200°C

### Beispiel 49:

0.1 g der Verbindung **X9j**, 0.09 g TBTU und 0.25 mL DIPEA wurden in 1.5 mL Dichlormethan gelöst und 30 Minuten bei 25°C gerührt. Dann wurden 0.02 mL Isopropylamin zugegeben und über Nacht bei 25°C gerührt. Die Reaktionsmischung wurde mit wässriger Kaliumcarbonat-Lösung gewaschen und die organische Phase eingeengt. Der Rückstand wurde in Aceton/Ether aufgenommen und das Produkt durch Zugabe von etherischer Salzsäure-Lösung gefällt.
Ausbeute: 0.07 g (farbloser Feststoff) Smp. 179-181 °C

### Beispiel 51:

0.1 g der Verbindung **X9j**, 0.09 g TBTU und 0.50 mL DIPEA wurden in 1.5 mL Dichlormethan gelöst und 30 Minuten bei 25°C gerührt. Dann wurden 0.07 g trans-4-Morphofino-cyclohexylamin zugegeben und über Nacht bei 25°C, gerührt. Die Reaktionsmischung wurde mit wässriger Kaliumcarbonat-Lösung gewaschen und die organische Phase eingeengt. Der Rückstand wurde durch Zugabe von Ether kristallisiert.
Ausbeute: 0.08 g (leicht gelber Feststoff) Smp. 166-168°C

### Beispiel 52:

0.1 g der Verbindung **X9j**, 0.09 g TBTU und 0.25 mL DIPEA wurden in 1.5 mL Dichlormethan gelöst und 30 Minuten bei 25°C gerührt. Dann wurden 0.03 g 1-Methylpiperidin-4-amin zugegeben und über Nacht bei 25°C gerührt. Die Reaktionsmischung wurde mit wässriger Kaliumcarbonat-Lösung gewaschen und die organische Phase eingeengt. Der Rückstand wurde durch Zugabe von Ether kristallisiert.
Ausbeute: 0.03 g (leicht gelber Feststoff) Smp. 148-151 °C

### trans-4-Morpholino-cyclohexylamin

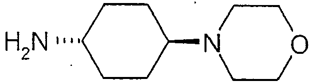

### Dibenzyl-4-morpholino-cyclohexylamin

3.9 g (30 mmol)) 4-Dibenzylcyclohexanon wurden in 100 mL CH₂Cl₂ gelöst und mit 3.9 g (45 mmol) Morpholin und 9,5 g (45 mmol) NaBH(OAc)₃ 12 Stunden bei 25°C gerührt. Anschließend wurde mit Wasser und Kaliumcarbonat versetzt, die organische Phase abgetrennt, getrocknet und eingeengt. Der Rückstand wurde über eine Kieselgelsäule gereinigt (Laufmittel: Essigester 90/ Methanol 10 + 1 % konz. Ammoniak). Die geeigneten Fraktionen wurden im Vakuum eingeengt.
Ausbeute: 6.6 g (60%) *cis*-Isomer und 2 g (18%) *trans*-Isomer.

Alternativ kann das *trans*-Dibenzyl-4-morpholino-cyclohexylamin nach folgendem Weg dargestellt werden:

33 g (112 mmol) 4-Dibenzylcyclohexanon wurden in 300 mL Methanol gelöst, mit 17.4 g (250 mmol) Hydroxylaminhydrochlorid versetzt und 4 Stunden bei 60°C gerührt. Das Lösungsmittel wurde im Vakuum eingeengt, mit 500 mL Wasser und 50 g Kaliumcarbonat versetzt und zwei Mal mit je 300 mL Dichlormethan extrahiert. Die organischen Phasen wurden getrocknet, im Vakuum eingeengt, der Rückstand aus Petrolether kristallisiert, in 1.5 L Ethanol gelöst und auf 70°C erwärmt. Es wurden 166 g Natrium portionsweise hinzugefügt und bis zur Auflösung des Natriums unter Rückfluss gekocht. Das Lösungsmittel wurde entfernt, der Rückstand mit 100 mL Wasser versetzt und zwei Mal mit je 400 mL Ether extrahiert. Die organischen Phasen wurden mit Wasser gewaschen, getrocknet, im Vakuum eingeengt und über eine Säule das *trans*-Isomer isoliert (Laufmittel: Essigester 80/ Methanol 20 + 2 % konz. Ammoniak).
Ausbeute: 12,6 g (41 %).

6.8 g (23 mmol) trans-1-Amino-4-dibenzylaminocyclohexan wurde in 90 mL DMF gelöst und mit 5 mL (42 mmol) 2,2'-Dichlorethylether und 5 g Kaliumcarbonat 8 Stunden bei 100°C gerührt. Nach Abkühlung wurde mit 30 mL Wasser versetzt, ausgefallene Kristalle abgesaugt und über eine kurze Säule (Laufmittel: Essigester) gereinigt. Der Rückstand wurde aus Methanol und konz. Salzsäure als Dihydrochlorid kristallisiert.
Ausbeute: 7.3 g (72%).

### trans-4-Morpholino-cyclohexylamin

7.2 g (16.4 mmol) *trans*-Dibenzyl-4-morpholino-cyclohexylamin wurden in 100 mL Methanol gelöst und an 1.4 g Pd/C (10%) bei 30-50 °C hydriert. Das Lösungsmittel wurde im Vakuum abgezogen und der Rückstand aus Ethanol und konz. Salzsäure kristallisiert.
Ausbeute: 3.9 g (93%); Smp. 312 °C.

Analog zu vorstehend beschriebener Vorgehensweise werden u.a. die in Tabelle 1 aufgeführten Verbindungen der Formel (I) erhalten.

**Tabelle 1**

| | | | | | | | |
|---|---|---|---|---|---|---|---|
| 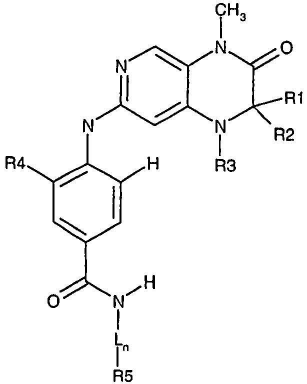 | | | | | | | |

| Beispiel | Konfig R1/R2 | R1 | R2 | R3 | R4 | Lₙ-R5 | Schmelzpunkt |
|---|---|---|---|---|---|---|---|
| 1 | rac | H | 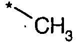 | 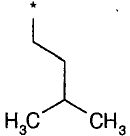 | H | 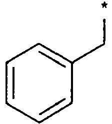 | 227 - 228°C Zersetzung |
| 2 | rac | H | 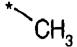 | 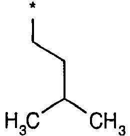 | H | 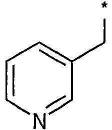 | 167 - 168°C Zersetzung |
| 3 | rac | H | 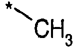 | 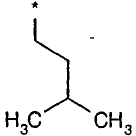 | H | 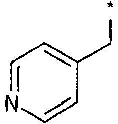 | 179 - 180°C Zersetzung |
| 4 | rac | H | 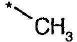 | 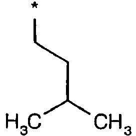 | 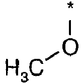 | 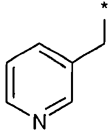 | 97 - 98°C Zersetzung |
| 5 | rac | H | 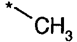 | 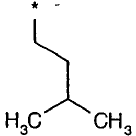 | 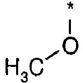 | 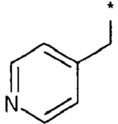 | |
| 6 | rac | H | 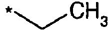 | 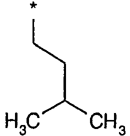 | 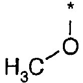 | 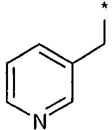 | 104 - 112°C |
| 7 | rac | H | 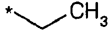 | 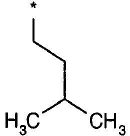 | 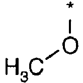 | 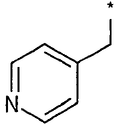 | 202 - 205°C |
| 8 | rac | H | 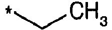 | 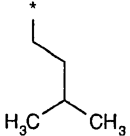 | 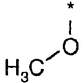 | 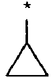 | 201 - 203°C |
| 9 | rac | H | 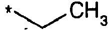 | 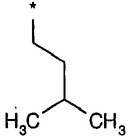 | 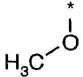 | H | 259 - 260°C |
| 10 | rac | H | 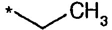 | 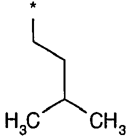 | 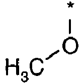 | 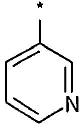 | |
| 11 | rac | H | 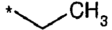 | 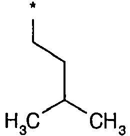 | 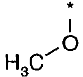 | 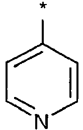 | |
| 12 | rac | H | 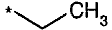 | 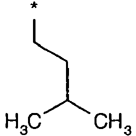 | H | 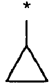 | 209 - 210°C |
| 13 | rac | H | 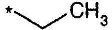 | 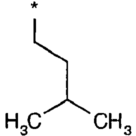 | H | 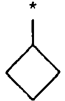 | 238 - 239°C |
| 14 | rac | H | 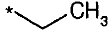 | 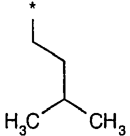 | H | 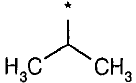 | 259 - 260°C |
| 15 | rac | H | 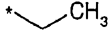 | 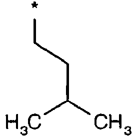 | H | 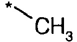 | 236 - 237°C |
| 16 | rac | H | 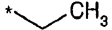 | 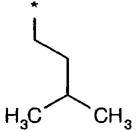 | H | 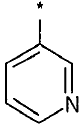 | 146 - 147°C Zersetzung |
| 17 | rac | H | 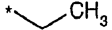 | 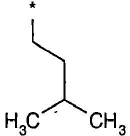 | H | 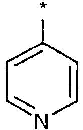 | 240 - 241 °C Zersetzung |
| 18 | | H | H | 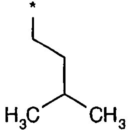 | H | 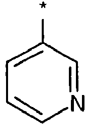 | 231 - 232°C Zersetzung |
| 19 | | 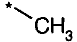 | 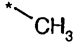 | 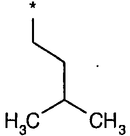 | H | 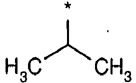 | 152°C |
| 20 | | 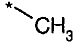 | 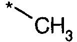 | 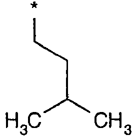 | H | 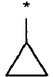 | 222°C |
| 21 | | 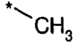 | 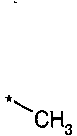 | 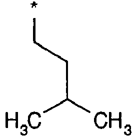 | H | H | 150°C |
| 22 | | 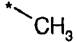 | 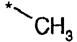 | 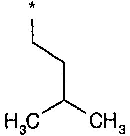 | H | 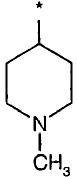 | |
| 23 | | 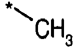 | 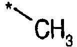 | 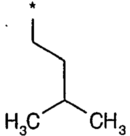 | H | 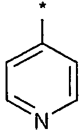 | 167°C |
| 24 | | 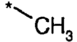 | 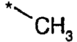 | 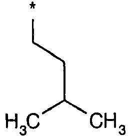 | H | 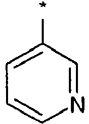 | 135°C |
| 25 | | 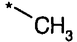 | 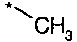 | 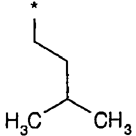 | 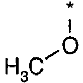 | 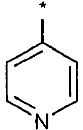 | 117°C |
| 26 | | 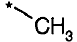 | 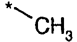 | 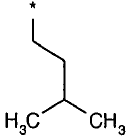 | 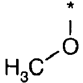 | 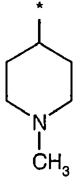 | 165°C |
| 27 | | 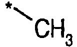 | 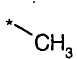 | 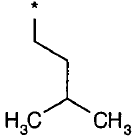 | 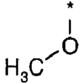 | 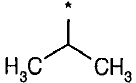 | 109°C |
| 28 | | 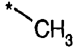 | 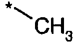 | 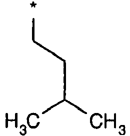 | 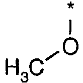 | H | 220°C |
| 29 | | 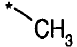 | 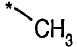 | 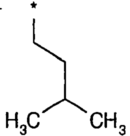 | 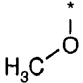 | 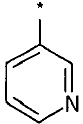 | |
| 30 | rac | H | 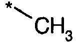 | 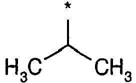 | H | 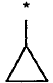 | 160°C |
| 31 | rac | H | 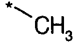 | 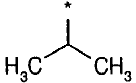 | H | 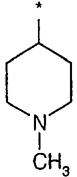 | 186 - 187°C |
| 32 | rac | H | 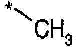 | 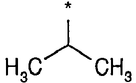 | H | 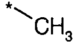 | 206 - 207°C |
| 33 | rac | H | 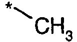 | 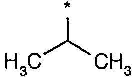 | H | 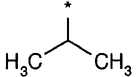 | 210 - 211°C |
| 34 | rac | H | 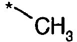 | 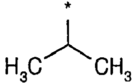 | H | H | |
| 35 | rac | H | 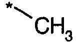 | 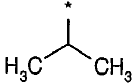 | H | 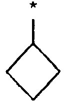 | |
| 36 | rac | H | 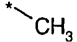 | 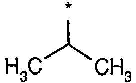 | H | 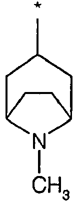 | >250°C |
| 37 | | 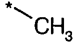 | 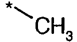 | 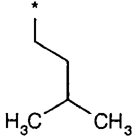 | 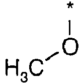 | 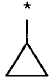 | 187°C |
| 38 | | 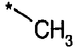 | 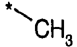 | 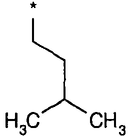 | 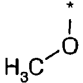 | 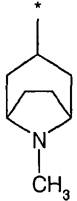 | 144°C |
| 39 | rac | H | 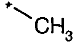 | 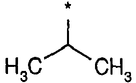 | 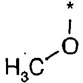 | 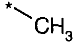 | 162 - 163°C |
| 40 | rac | H | 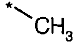 | 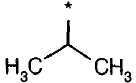 | 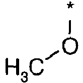 | 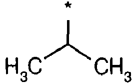 | 188 - 189°C |
| 41 | rac | H | 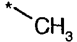 | 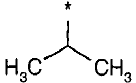 | 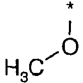 | 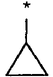 | 201 - 203°C |
| 42 | rac | H | 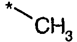 | 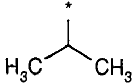 | 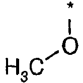 | 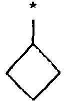 | 191 - 192°C |
| 43 | rac | H | 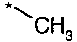 | 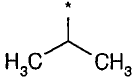 | 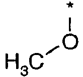 | 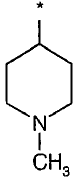 | 160 - 161°C |
| 44 | rac | H | 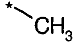 | 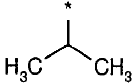 | 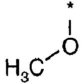 | 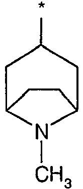 | > 200°C |
| 45 | rac | H | 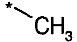 | 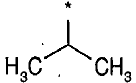 | 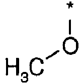 | H | > 200°C |
| 46 | rac | H | 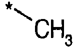 | 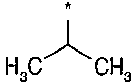 | H | 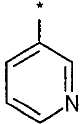 | > 200°C |
| 47 | rac | H | 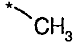 | 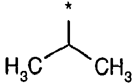 | 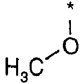 | 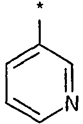 | |
| A8 | R | H | 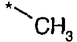 | 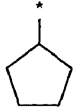 | 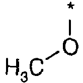 | 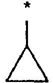 | |
| 49 | R | H | 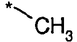 | 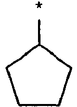 | 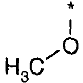 | 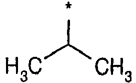 | 179 - 181°C |
| 50 | R | H | 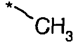 | 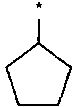 | 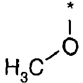 | 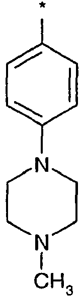 | |
| 51 | R | H | 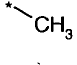 | 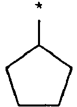 | 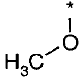 | 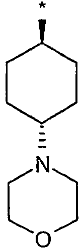 | 166 - 168 °C |
| 52 | R | H | 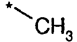 | 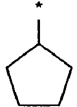 | 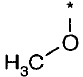 | 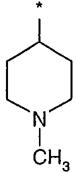 | 148 -151 °C |
| 53 | R | H | 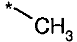 | 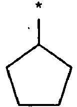 | 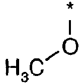 | H | 140 -142 °C |
| 54 | R | H | 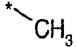 | 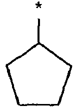 | 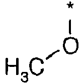 | 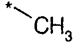 | 179 -181 °C |
| 55 | R | H | 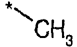 | 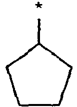 | 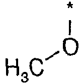 | 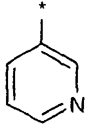 | |

Wie gefunden wurde, zeichnen sich die Verbindungen der allgemeinen Formel (1) durch vielfältige Anwendungsmöglichkeiten auf therapeutischem Gebiet aus. Hervorzuheben sind solche Anwendungsmöglichkeiten, für welche die Inhibition spezifischer Zellzykluskinasen, insbesondere die inhibierende Wirkung auf die Proliferation kultivierter humaner Tumorzellen, aber auch auf die Proliferation anderer Zellen, wie z.B. Endothelzellen, eine Rolle spielen.

Wie durch DNA-Färbung mit darauf folgender FACS Analyse gezeigt werden konnte, ist die, durch die erfindungsgemäßen Verbindungen bewirkte, Proliferationsinhibition vermittelt durch einen Arrest der Zellen vor allem in der G2/M Phase des Zellzyklus. Die Zellen arretieren abhängig von den verwendeten Zellen für eine bestimmte Zeitspanne in dieser Zellzyklus Phase, bevor der programmierte Zelltod eingeleitet wird. Ein Arrest in der G2/M Phase des Zellzyklus wird z.B. durch die Inhibition spezifischer Zellzykluskinasen ausgelöst. Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut-und Progenitor-Zellen) gegen DNA-Schädigung durch Strahlung, UV-Behandlung und/oder zytostatischer Behandlung (Davis et al., 2001).

Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Cytostatika, Hormone oder Antikörper verwendet werden.

Die Wirkung der erfindungsgemäßen Verbindungen wurde im PLK1 Inhibitionsassay, im Cytotoxizitätstest an kultivierten humanen Tumorzellen und/oder in einer FACS-Analyse, beispielsweise an HeLa S3-Zellen, bestimmt. Die Verbindungen zeigten in beiden Testmethoden eine gute bis sehr gute Wirksamkeit, d.h. beispielsweise einen EC₅₀-Wert im HeLa S3-Cytotoxizitätstest kleiner 5 µmol/L, in der Regel kleiner 1 µmol/L und einen IC₅₀-Wert im PLK1-Inhibitionsassay kleiner 1 µmol/L.

### PLK1 Kinaseassay

### Enzymherstellung:

Rekombinantes humanes und an seinem N-terminalen Ende mit GST verbundenes PLK1 Enzym wird aus Bakulovirus infizierten Insektenzellen (Sf21) isoliert. Die Reinigung erfolgt durch Affinitätschromatographie an Glutathion Sepharose Säulen.

4x10⁷ Sf21 Zellen (Spodoptera frugiperda) in 200 ml Sf-900 II Serum freien Insektenzellmedium (Life Technologies) werden in eine Spinnerflasche ausgesät. Nach 72 Stunden Inkubation bei 27°C und 70 rpm werden 1x10⁸ Sf21 Zellen in insgesamt 180 ml Medium in eine neue Spinnerflasche ausgesät. Nach weiteren 24 Stunden werden 20 ml rekombinanter Baculovirus Stammsuspension zugesetzt und die Zellen 72 Stunden bei 27°C bei 70 rpm kultiviert. 3 Stunden vor dem Ernten wird Okadainsäure zugesetzt (Calbiochem, Endkonzentration 0,1 µM) und die Suspension weiter inkubiert. Die Zellzahl wird bestimmt, die Zellen abzentrifugiert (5 Minuten, 4°C, 800 rpm) und 1x mit PBS (8 g NaCl/l, 0,2 g KCl/l, 1,44 g Na₂HPO₄/l, 0,24 g KH₂PO4/l) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7,5, 10 mM MgCl₂, 1 mM DTT, 5 µg/ml Leupeptin, 5 µg/ml Aprotinin, 100 µM NaF, 100 µM PMSF, 10 mM ß-Glycerolphosphat, 0.1 mM Na₃VO₄, 30 mM 4-Nitrophenylphosphate) zu 1x10⁸ Zellen/ 17,5 ml resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 ml resuspendierte und gewaschene Beads für 50 ml Überstand) und 30 Minuten bei 4°C auf einem Rotationsbrett inkubiert. Danach werden die Beads mit Lysispuffer gewaschen und das rekombinante Protein mit 1 ml Elutionspuffer/ ml resuspendierte Beads (Elutionspuffer: 100 mM Tris/HCl pH=8,0, 120 mM NaCl, 20 mM reduziertes Glutathion (Sigma G-4251), 10 mM MgCl₂, 1 mM DTT) von den Beads eluiert. Die Proteinkonzentration wird mittels Bradford Assay bestimmt.

### Assaydurchführung:

In einem Napf einer 96-Loch Rundbodenplatte (Fa. Greiner bio-one, PS-Microtiterplatte Nr.650101) werden folgende Komponenten zusammengefügt:
- 10 µl zu testende Verbindung in variabler Konzentration (z.B. beginnend bei 300 µM, und Verdünnung in 1:3) in 6% DMSO, 0,5 mg/ml Casein (Sigma C-5890), 60 mM ß-Glycerophosphat, 25 mM MOPS pH=7,0, 5 mM EGTA, 15 mM MgCl₂, 1 mM DTT
- 20 µl Substratlösung (25 mM MOPS pH=7,0, 15 mM MgCl₂, 1 mM DTT, 2,5 mM EGTA, 30 mM ß-Glycerophosphat, 0,25 mg/ml Casein)
- 20 µl Enzymverdünnung (1:100 Verdünnung des Enzymstocks in 25 mM MOPS pH=7,0, 15 mM MgCl₂, 1 mM DTT)
- 10 µl ATP Lösung (45 µM ATP mit 1,11 x10⁶ Bq/ml gamma-P33-ATP).

Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µl eiskalter 5%iger TCA pro Napf gestoppt und mindestens 30 Minuten auf Eis inkubiert. Das Präziptitat wird durch Ernten auf Filterplatten (96-well-Microtiter-Filterplatte: UniFilter-96, GF/B; Fa. Packard; Nr.6005177) übertragen, dann viermal mit 1 %iger TCA gewaschen und bei 60°C getrocknet. Nach Zugabe von 35µl Szintillationslösung (Ready-Safe; Beckmann) pro Napf wird die Platte mit Sealing-tape zugeklebt und die präzipitierte Menge P33 mit dem Wallac Betacounter gemessen.

Die Messdaten werden mit der Standard Graphpad Software (Levenburg-Marquard Algorhythmus) ausgewertet.

### Messung der Cytotoxizität an kultivierten humanen Tumorzellen

Zur Messung der Cytotoxizität an kultivierten humanen Tumorzellen wurden Zellen der zervikalen Carcinoma Tumorzell-Linie HeLa S3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend wurden die HeLa S3 Zellen in 96-well Platten (Costar) mit einer Dichte von 1000 Zellen pro weil eingebracht und über Nacht in einem Inkubator (bei 37°C und 5 % CO₂) inkubiert, wobei auf jeder Platte 6 wells nur mit Medium gefüllt wurden (3 wells zur Mediumkontrolle, 3 wells zur Inkubation mit reduzierten AlamarBlue Reagenz). Die Wirksubstanzen wurden in verschiedenen Konzentrationen (gelöst in DMSO; DMSO-Endkonzentration: 0.1 %) zu den Zellen zugegeben (jeweils als Dreifachbestimmung). Nach 72 Stunden Inkubation wurden zu jeden well 20 µl AlamarBlue Reagenz (AccuMed International) zugesetzt, und die Zellen für weitere 7 Stunden inkubiert. Zur Kontrolle wurde zu 3 wells je 20 µl reduziertes AlamarBlue Reagenz gegeben (AlamarBlue Reagenz, das für 30 min autoklaviert wurde). Nach 7 h Inkubation wurde der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wurde in Prozent der Kontrolle (HeLa S3 Zellen ohne Inhibitor) berechnet und die Wirkstoffkonzentration, die die Zellaktivität zu 50% hemmt (IC₅₀) abgeleitet. Die Werte wurden hierbei aus dem Mittelwert von drei Einzelbestimmungen - unter Korrektur des Leerwertes (Mediumkontrolle)- berechnet.

### FACS- Analyse

Propidium lodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.

Für eine PI-Färbung wurden beispielsweise 0.4 Mio HeLa S3 Zellen auf eine 75 cm² Zellkulturflasche ausgesät, nach 24 h wurde entweder 0.1 % DMSO als Kontrolle zugesetzt, bzw. die Substanz in verschiedenen Konzentrationen (in 0.1 % DMSO). Die Zellen wurden für 24 h mit der Substanz bzw. mit DMSO inkubiert, bevor die Zellen 2 x mit PBS gewaschen und dann mit Trypsin /EDTA abgelöst wurden. Die Zellen wurden zentrifugiert (1000 Upm, 5 min, 4°C), und das Zellpellet 2 x mit PBS gewaschen, bevor die Zellen in 0.1 ml PBS resuspendiert wurden. Anschließend wurden die Zellen für 16 Stunden bei 4°C oder alternativ für 2 Stunden bei -20°C mit 80% Ethanol fixiert. Die fixierten Zellen (10⁶ Zellen) wurden zentrifugiert (1000 Upm, 5min, 4°C), mit PBS gewaschen und anschließend nochmals zentrifugiert. Das Zellpellet wurde in 2 ml Triton X-100 in 0.25 % PBS resuspendiert, und 5 min auf Eis inkubiert, bevor 5 ml PBS zugeben wurden und erneut zentrifugiert wurde. Das Zellpellet wurde in 350 µl Pl Färbelösung (0.1 mg/ml RNase A, 10 µg/ml Prodium lodid in 1 x PBS) resuspendiert. Die Zellen wurden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einen Argonlaser (500 mW, Emission 488 nm), und dem DNA Cell Quest Programm (BD). Die logarithmische Pl Fluoreszenz wurde mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Entsprechend, wurden erfindungsgemäße Verbindungen auf weiteren Tumorzellen getestet. Beispielsweise sind diese Verbindungen auf Karzinomen verschiedenster Gewebe (z. Bspl. Brust (MCF7); Colon (HCT116), Kopf-Hals (FaDu), Lunge (NCl-H460), Pankreas (BxPC-3), Prostata (DU145)), Sarkome (z. Bspl. SK-UT-1 B), Leukämien und Lymphome (z. Bspl. HL-60; Jurkat, THP-1) und anderen Tumoren (z. Bspl. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehütte zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Die Dosierung für die intravenöse Anwendung liegt bei 1 - 1000 mg pro-Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feuchtgranuliert und getrocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen entsprechend der allgemeinen Formel (I), 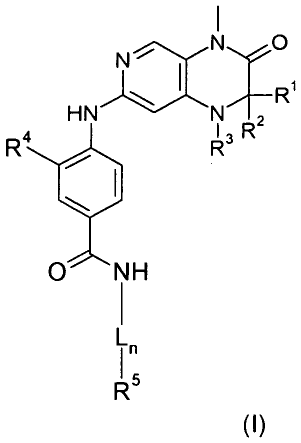 worin
R¹, R² gleich oder verschieden, Wasserstoff oder ein Rest, ausgewählt aus der Gruppe bestehend aus gegebenenfalls substituiertem C₁-C₆-Alkyl, C₂-C₆-Alkenyl, und C₂-C₆-Alkinyl,
oder
R¹ und R² gemeinsam eine 2- bis 5-gliedrige Alkylbrücke,
R³ Wasserstoff oder ein Rest, ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktem C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl,
R⁴ ein Rest, ausgewählt aus der Gruppe bestehend aus Wasserstoff, Hydroxy und Halogen, oder
ein Rest ausgewählt aus der Gruppe bestehend aus gegebenenfalls substituiertem C₁-C₃-Alkyl, C₂-C₆-Alkenyl, C₂-C₆-Alkinyl, C₁-C₅-Alkyloxy, C₂-C₅-Alkenyloxy und C₂-C₅-Alkinyloxy,
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₁-C₄-Alkyl-C₆-C₁₄-Aryl , C₆-C₁₄-Heteroaryl, und gegebenenfalls verbrücktem C₃-C₁₂-Cycloalkyl,
n 0 oder 1
R⁵ ein Rest, ausgewählt aus der Gruppe bestehend aus Wasserstoff oder gegebenenfalls substituiertem C₁-C₆-Alkyl, C₁-C₆-Alkenyl, C₁-C₆-Alkinyl, C₃-C₁₂-Cycloalkyl, oder
ein Rest, ausgewählt aus der Gruppe bestehend aus gegebenenfalls substituiertem Pyridyl, Morpholinyl, Piperidinyl, Piperazinyl, Piperazinylcarbonyl, Pyrrolidinyl, Tropenyl, Sulfoxomorpholinyl, Sulfonylmorpholinyl, Thiomorpholinyl und Azacycloheptyl,
wobei die Substituenten der gegebenenfalls substituiertem Alkylgruppen, soweit nicht anders beschrieben, ausgewählt sein können aus ein oder mehreren Fluoratomen, wobei die Substituenten der gegebenenfalls substituiertem Arylgruppen, soweit nicht anders beschrieben, ausgewählt sein können aus -OH, -NO₂, -CN, -OMe, -OCHF₂, - OCF₃, -NH₂, oder Halogen,
wobei die Substituenten der gegebenenfalls substituiertem Cycloalkylgruppen, soweit nicht anders beschrieben, ausgewählt sein können aus -OH, -NO₂, -CN, -OMe, - OCHF₂, -OCF₃, -NH₂, oder Halogen,
wobei die Substituenten der gegebenenfalls substituiertem Heteroarylgruppen, soweit nicht anders beschrieben, ausgewählt sein können aus -OH, -NO₂, -CN, - OMe, -OCHF₂, -OCF₃, -NH₂, oder Halogen,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate oder Hydrate,
bedeuten.

2. Verbindungen nach Anspruch 1, worin
R³ bis R⁵, n und L die angegebene Bedeutung aufweisen, und
R¹, R² gleich oder verschieden, Wasserstoff , oder
ein Rest ausgewählt aus der Gruppe bestehend Methyl, Ethyl, Propyl, Propargyl und Allyl,
oder
R¹ und R² gemeinsam Cyclopropyl,
bedeuten.

3. Verbindungen nach Anspruch 1 oder 2, worin
R¹ bis R⁴ , n und L die angegebene Bedeutung aufweisen, und
R⁵ ein Rest ausgewählt aus der Gruppe bestehend aus Wasserstoff oder gegebenenfalls substituiertem C₁-C₆-Alkyl, C₃-C₁₂-Cycloalkyl,
oder ein Rest, ausgewählt aus der Gruppe bestehend aus gegebenenfalls substituiertem Pyridyl, Morpholinyl, Piperidinyl, Piperazinyl und Piperazinylcarbonyl, bedeutet.

4. Verbindungen nach einem der Ansprüche 1 bis 3, worin
R¹, R², R⁴, R⁵, n und L die angegebene Bedeutung aufweisen, und
R³ Wasserstoff, gegebenenfalls substituiertes C₁-C₆-Alkyl oder gegebenenfalls substituiertes und/oder verbrücktes C₃-C₁₂-Cycloalkyl,
bedeutet.

5. Verbindungen nach einem der Ansprüche 1 bis 4, worin
R¹, R², R³, R⁵, n und L die angegebene Bedeutung aufweisen, und
R⁴ ein Rest ausgewählt aus der Gruppe bestehend aus Wasserstoff, Hydroxy, Halogen, Methyl, Ethyl, Propinyloxy und Methoxy,
bedeutet.

6. Verbindung der Formel I nach einem der Ansprüche 1 bis 5 zur Verwendung als Arzneimittel.

7. Verbindung der Formel I nach einem der Ansprüche 1 bis 5 zur Verwendung als Arzneimittel mit antiproliferativer Wirkung.

8. Verwendung einer Verbindung der Formel I gemäß einem der Ansprüche 1 bis 5 zur Herstellung eines Arzneimittels zur Behandlung und/oder Prävention von Krebs, Infektionen, Entzündungs- und Autoimmunerkrankungen.

9. Pharmazeutische Zubereitungen, enthaltend als Wirkstoff eine oder mehrere Verbindungen der allgemeinen Formel (I) gemäß einem der Ansprüche 1 bis 5 oder deren physiologisch verträgliche Salze gegebenenfalls in Kombination mit üblichen Hilfs- und/oder Trägerstoffen.

10. Verfahren zur Herstellung einer Verbindung der allgemeinen Formel (I), 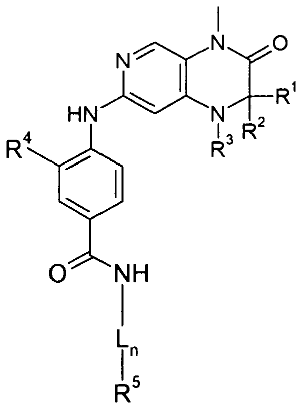 worin
R¹-R⁵, n und L die in den Ansprüchen 1 bis 5 angegebene Bedeutung aufweisen, **dadurch gekennzeichnet, dass** eine Verbindung der allgemeinen Formel (II) 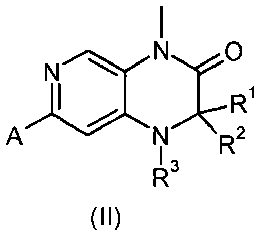 worin
R¹-R³ die in den Ansprüchen 1 bis 4 angegebene Bedeutung aufweisen und A eine Abgangsgruppe, ausgewählt aus -O-Methyl, -SCN, Fluor, Chlor, Brom, lod, Methansulfonyl, Trifluormethansulfonyl oder p-Toluolsulfonyl, ist,
mit einer gegebenenfalls substituierten Verbindung der allgemeinen Formel (III), 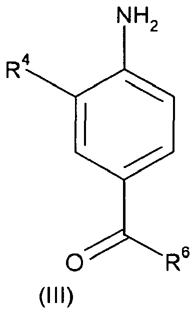 worin
R⁴ die in den Ansprüchen 1 bis 5 angegebene Bedeutung aufweist und R⁶ OH, -O-Methyl, -O-Ethyl bedeutet,
unter Erhalt eines Produkts der allgemeinen Formel (IV) umgesetzt wird, 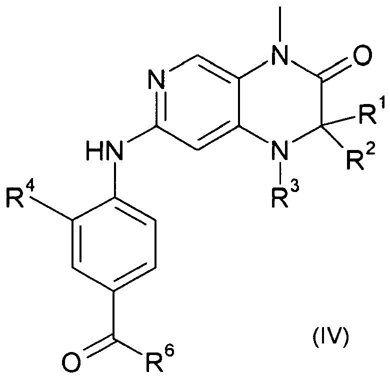 worin
R¹ bis R⁴ die in den Ansprüchen 1 bis 5 angegebene Bedeutung aufweist und
R⁶ OH, NH₂-Lₙ-R⁵, -O-Methyl oder -O-Ethyl bedeutet,
und gegebenenfalls anschliessend das erhaltene Produkt der allgemeinen Formel (IV) gegebenenfalls nach vorhergehender Hydrolyse der Estergruppe -COR⁶, mit einem Amin der allgemeinen Formel (V)
NH₂-Lₙ-R⁵ (V)
worin
R⁵ die in den Ansprüchen 1 bis 5 angegebene Bedeutung aufweist,
umgesetzt wird.

11. Verbindung der Formel (II), 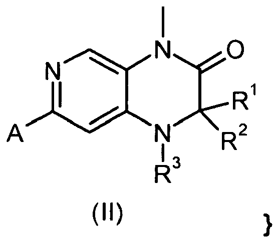 worin R¹-R³ die in den Ansprüchen 1 bis 5 angegebene Bedeutung aufweisen und A eine Abgangsgruppe ist

## Claims

1. Compounds corresponding to general formula (I), 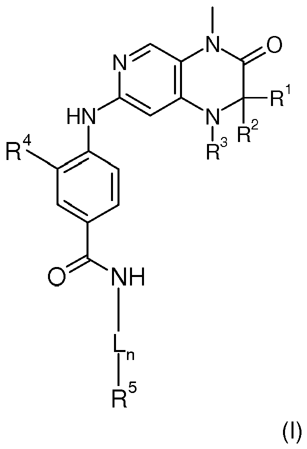 wherein
R¹, R² which may be identical or different denote hydrogen or a group selected from among optionally substituted C₁-C₆-alkyl, C₂-C₆-alkenyl and C₂-C₆-alkynyl,
or
R¹ and R² together denote a 2- to 5-membered alkyl bridge,
R³ denotes hydrogen or a group selected from among optionally substituted C₁-C₁₂-alkyl, C₂-C₁₂-alkenyl, C₂-C₁₂-alkynyl and C₆-C₁₄-aryl, or
a group selected from among optionally substituted and/or bridged C₃-C₁₂-cycloalkyl, C₃-C₁₂-cycloalkenyl, C₇-C₁₂-polycycloalkyl, C₇-C₁₂-polycycloalkenyl and C₅-C₁₂-spirocycloalkyl,
R⁴ denotes a group selected from among hydrogen, hydroxy and halogen, or
a group selected from among optionally substituted C₁-C₃-alkyl, C₂-C₆-alkenyl, C₂-C₆-alkynyl, C₁-C₅-alkyloxy, C₂-C₅-alkenyloxy and C₂-C₅-alkynyloxy,
L denotes a linker selected from among optionally substituted C₂-C₁₀-alkyl, C₂-C₁₀-alkenyl, C₆-C₁₄-aryl, -C₁-C₄-alkyl-C₆-C₁₄-aryl, C₆-C₁₄-heteroaryl, and optionally bridged C₃-C₁₂-cycloalkyl,
n denotes 0 or 1,
R⁵ denotes a group selected from among hydrogen or optionally substituted C₁-C₆-alkyl, C₁-C₆-alkenyl, C₁-C₆-alkynyl, C₃-C₁₂-cycloalkyl, or
a group selected from among optionally substituted pyridyl, morpholinyl, piperidinyl, piperazinyl, piperazinylcarbonyl, pyrrolidinyl, tropenyl, sulphoxomorpholinyl, sulphonylmorpholinyl, thiomorpholinyl and azacycloheptyl,
wherein the substituents of the optionally substituted alkyl groups, unless stated otherwise, may be selected from one or more fluorine atoms,
wherein the substituents of the optionally substituted aryl groups, unless stated otherwise, may be selected from -OH, -NO₂, -CN, -OMe, -OCHF₂, -OCF₃, -NH₂, or halogen,
wherein the substituents of the optionally substituted cycloalkyl groups, unless stated otherwise, may be selected from -OH, -NO₂, -CN, -OMe, -OCHF₂, -OCF₃, -NH₂, or halogen,
wherein the substituents of the optionally substituted heteroaryl groups, unless stated otherwise, may be selected from -OH, -NO₂, -CN, -OMe, -OCHF₂, -OCF₃, -NH₂, or halogen,
optionally in the form of the tautomers, the racemates, the enantiomers, the diastereomers and the mixtures thereof, and optionally the pharmacologically acceptable acid addition salts, solvates or hydrates thereof.

2. Compounds according to claim 1, wherein
R³ to R⁵, n and L are as hereinbefore defined, and
R¹, R², which may be identical or different, denote hydrogen, or
a group selected from among methyl, ethyl, propyl, propargyl and allyl,
or
R¹ and R² together represent cyclopropyl.

3. Compounds according to claim 1 or 2, wherein
R¹ to R⁴, n and L are as hereinbefore defined, and
R⁵ denotes a group selected from among hydrogen or optionally substituted C₁-C₆-alkyl, C₃-C₁₂-cycloalkyl,
or a group selected from among optionally substituted pyridyl, morpholinyl, piperidinyl, piperazinyl and piperazinylcarbonyl.

4. Compounds according to one of claims 1 to 3, wherein
R¹, R², R⁴, R⁵, n and L are as hereinbefore defined, and
R³ denotes hydrogen, optionally substituted C₁-C₆-alkyl or optionally substituted and/or bridged C₃-C₁₂-cycloalkyl.

5. Compounds according to one of claims 1 to 4, wherein
R¹, R², R³, R⁵, n and L are as hereinbefore defined, and
R⁴ denotes a group selected from among hydrogen, hydroxy, halogen, methyl, ethyl, propynyloxy and methoxy.

6. Compound of formula I according to one of claims 1 to 5 for use as a medicament.

7. Compound of formula I according to one of claims 1 to 5 for use as a medicament with an antiproliferative activity.

8. Use of a compound of formula I according to one of claims 1 to 5 for preparing a medicament for the treatment and/or prevention of cancer, infections, inflammatory and autoimmune diseases.

9. Pharmaceutical preparations, containing as active substance one or more compounds of general formula (I) according to one of claims 1 to 5 or the physiologically acceptable salts thereof, optionally combined with conventional excipients and/or carriers.

10. Process for preparing a compound of general formula (I), 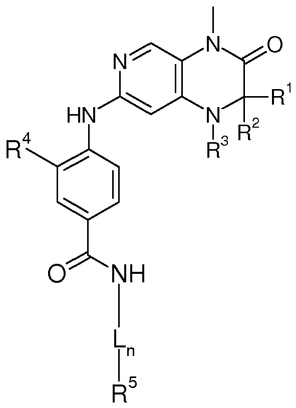 wherein
R¹-R⁵, n and L have the meanings given in claims 1 to 5,
**characterised in that** a compound of general formula (II) 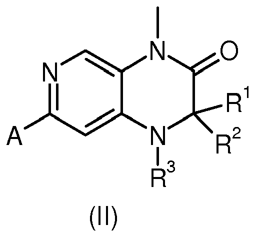 wherein
R¹-R³ have the meanings given in claims 1 to 4 and A is a leaving group selected from among -O-methyl, -SCN, fluorine, chlorine, bromine, iodine, methanesulphonyl, trifluoromethanesulphonyl or p-toluenesulphonyl,
is reacted with an optionally substituted compound of general formula (III), 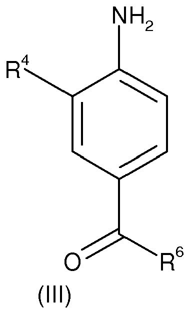 wherein
R⁴ has the meanings given in claims 1 to 5 and R⁶ denotes OH, -O-methyl, -O-ethyl,
to obtain a product of general formula (IV), 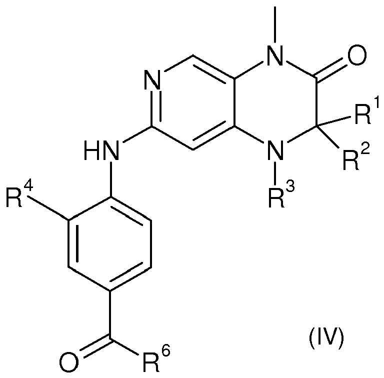 wherein
R¹ to R⁴ have the meanings given in claims 1 to 5 and
R⁶ denotes OH, NH₂-Lₙ-R⁵, -O-methyl or -O-ethyl,
and then the product of general formula (IV) obtained, optionally after previous hydrolysis of the ester group -COR⁶, is optionally reacted with an amine of general formula (V)
NH₂-Lₙ-R⁵ (V)
wherein
R⁵ has the meanings given in claims 1 to 5.

11. Compound of formula (II), 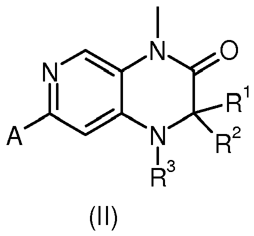 wherein
R¹-R³ have the meanings given in claims 1 to 5 and A is a leaving group.

## Revendications

1. Composés correspondant à la formule générale (I) 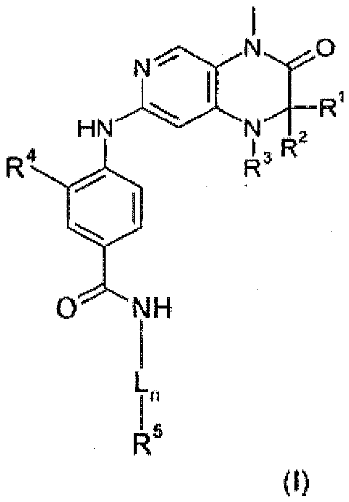 dans lesquels
R¹, R², identiques ou différents, représentent l'hydrogène ou un radical choisi dans le groupe comprenant les groupes alkyle en C₁ à C₆ éventuellement substitué, alcényle en C₂ à C₆ et alcynyle en C₂ à C₆,
ou
R¹ et R² représentent conjointement un pont alkyle de 2 à 5 chaînons,
R³ représente l'hydrogène ou un radical choisi dans le groupe comprenant les groupes alkyle en C₁ à C₁₂ éventuellement substitué, alcényle en C₂ à C₁₂, alcynyle en C₂ à C₁₂ et aryle en C₆ à C₁₄,
ou
un radical choisi dans le groupe comprenant les groupes cycloalkyle en C₃ à C₁₂ éventuellement substitués et/ou pontés, cycloalcényle en C₃ à C₁₂, polycycloalkyle en C₇ à C₁₂, polycyclo-alcényle en C₇ à C₁₂ et spirocycloalkyle en C₅ à C₁₂,
R⁴ représente un radical choisi dans le groupe comprenant l'hydrogène, un groupe hydroxyle et halogéno, ou
un radical choisi dans le groupe comprenant les groupes alkyle en C₁ à C₃ éventuellement substitués, alcényle en C₂ à C₆, alcynyle en C₂ à C₆, alkyloxy en C₁ à C₅, alcényloxy en C₂ à C₅ et alcinyloxy en C₂ à C₅,
L est un segment de liaison choisi dans le groupe comprenant les groupes alkyle en C₂ à C₁₀ éventuellement substitués, alcényle en C₂ à C₁₀, aryle en C₆ à C₁₄, alkyle en C₁ à C₄-aryle en C₆ à C₁₄, hétéroaryle en C₆ à C₁₄ et cycloalkyle en C₃ à C₁₂ éventuellement pontés,
n est 0 ou 1
R⁵ est un radical choisi dans le groupe comprenant l'hydrogène ou les groupes alkyle en C₁ à C₆ éventuellement substitués, alcényle en C₁ à C₆, alcynyle en C₁ à C₆, cycloalkyle en C₃ à C₁₂, ou un radical choisi dans le groupe comprenant les groupes pyridyle éventuellement substitués, morpholinyle, pipéridinyle, pipérazinyle, pipérazinylcarbonyle, pyrrolidinyle, tropényle, sulfoxomorpholinyle, sulfonylmorpholinyle, thiomorpholinyle et azacycloheptyle,
dans lesquels les substituants des groupes alkyle éventuellement substitués peuvent être choisis, sauf indication contraire, parmi un ou plusieurs atomes de fluor,
dans lesquels les substituants des groupes aryle éventuellement substitués peuvent être choisis, sauf indication contraire, parmi -OH, -NO₂, -CN, -OMe, -OCHF₂-, OCF₃, -NH₂ ou halogéno,
dans lesquels les substituants des groupes cycloalkyle éventuellement substitués peuvent être choisis, sauf indication contraire, parmi -OH, -NO₂, -CN, -OMe, -OCHF₂, -OCF₃, -NH₂ ou halogéno, dans lesquels les substituants des groupes hétéroaryle éventuellement substitués peuvent être choisis, sauf indication contraire, parmi -OH, -N0₂, -CN, -OMe, -OCHF₂, -OCF₃, -NH₂ ou halogéno, éventuellement sous la forme de leurs tautomères, leurs racémates, leurs énantiomères, leurs dias-téréomères et leurs mélanges, et éventuellement leurs sels d'addition acides, solvates ou hydrates pharmacologiquement acceptables.

2. Composés selon la revendication 1, dans lesquels
R³ à R⁵, n et L présentent la signification indiquée, et
R¹, R², identiques ou différents, représentent l'hydrogène, ou
un radical choisi dans le groupe comprenant les groupes méthyle, éthyle, propyl, propargyle et allyle
ou
R¹ et R² représentent conjointement le groupe cyclo-propyle.

3. Composés selon la revendication 1 ou 2, dans lesquels
R¹ à R⁴, n et L présentent la signification indiquée, et
R⁵ représente un radical choisi dans le groupe comprenant l'hydrogène ou les groupes alkyle en C₁ à C₆ éventuellement substitués, cycloalkyle en C₃ à C₁₂
ou un radical choisi dans le groupe comprenant les groupes pyridyle, morpholinyle, pipéridinyle, pipérazinyle et pipérazinylcarbonyle éventuellement substitués.

4. Composés selon l'une quelconque des revendications 1 à 3, dans lesquels
R¹, R², R⁴, R⁵, n et L présentent la signification indiquée, et
R³ représente l'hydrogène, un groupe alkyle en C₁ à C₆ éventuellement substitué ou un groupe cycloalkyle en C₃ à C₁₂ éventuellement substitué et/ou ponté.

5. Composés selon l'une quelconque des revendications 1 à 4, dans lesquels
R¹, R², R³, R⁵, n et L présentent la signification indiquée, et
R⁴ représente un radical choisi dans le groupe comprenant l'hydrogène, les groupes hydroxy, halogéno, méthyle, éthyle, propinyloxy et méthoxy.

6. Composé de formule I selon l'une quelconque des revendications 1 à 5 pour son utilisation comme médicament.

7. Composé de formule I selon l'une quelconque des revendications 1 à 5, pour son utilisation comme médicament ayant un effet antiprolifératif.

8. Utilisation d'un composé de formule I selon l'une quelconque des revendications 1 à 5, pour la production d'un médicament pour le traitement et/ou la prévention du cancer, d'infections, de maladies inflammatoires et auto-immunes.

9. Préparations pharmaceutiques, contenant comme substance active, un ou plusieurs composés de formule générale (I) selon l'une quelconque des revendications 1 à 5, ou leurs sels physiologiquement acceptables, éventuellement en combinaison avec des adjuvants et/ou véhicules habituels.

10. Procédé de production d'un composé de formule générale (I),
dans lequel
R¹ à R⁵, n et L présentent la signification indiquée dans les revendications 1 à 5,
**caractérisé en ce qu'**on fait réagir un composé de formule générale (II) 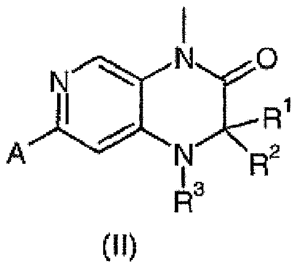 dans lequel
R¹ à R³ présentent la signification indiquée dans les revendications 1 à 4 et A est un groupe partant, choisi parmi les groupes -O-méthyle, -SCN, fluoro, chloro, bromo, iodo, méthane-sulfonyle, trifluorométhanesulfonyle ou p-toluènesulfonyle,
avec un composé éventuellement substitué de formule générale (III) 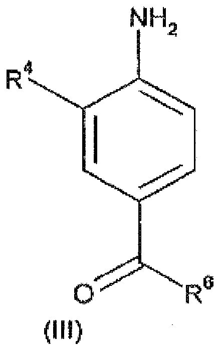 dans lequel
R⁴ présente la signification indiquée dans les revendications 1 à 5 et
R⁶ représente les groupes OH, -O-méthyle, -O-éthyle, donnant un produit de formule générale (IV) 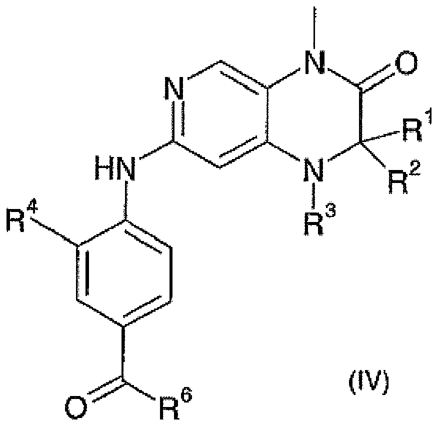 dans lequel
R¹ à R⁴ présentent la signification indiquée dans les revendications 1 à 5 et
R⁶ représente les groupes OH, NH₂-Lₙ-R⁵, -O-méthyle ou -O-éthyle,
et éventuellement, le produit de formule générale (IV) obtenu éventuellement après hydrolyse préalable du groupe ester -COR⁶ est ensuite converti avec une amine de formule générale (V)
NH₂-Lₙ-R⁵ (V)
dans laquelle
R⁵ présente la signification indiquée dans les revendications 1 à 5.

11. Composé de formule (II) 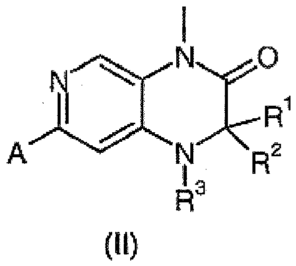 dans lequel
R¹ à R³ présentent la signification indiquée dans les revendications 1 à 5 et A est un groupe partant.
